# EUROPEAN PATENT SPECIFICATION

(11) **EP 4 308 572 B1**
(45) Date of publication and mention of the grant of the patent: **23.04.2025**
(21) Application number: 22715022.4
(22) Date of filing: 15.03.2022
(51) Int. Cl.: C07D 495/04, A61K 31/4365, A61P 25/28

(54) **NEW THIENOPYRIMIDINONE DERIVATIVES**
THIENOPYRIMIDINONDERIVATE
NOUVEAUX DÉRIVÉS DE THIÉNOPYRIMIDINONE

(30) Priority: 17.03.2021 EP 21163258
(43) Date of publication of application: 24.01.2024
(73) Proprietor: F. Hoffmann-La Roche AG, 4070 Basel (CH)
(72) Inventor: DOLENTE, Cosimo, 4070 Basel (CH); GRETHER, Nadine, 4070 Basel (CH); O'HARA, Fionn Susannah, 4070 Basel (CH); PIRAS, Matilde, 4070 Basel (CH); RATNI, Hasane, 4070 Basel (CH); REUTLINGER, Michael, 4070 Basel (CH); VIFIAN, Walter, 4070 Basel (CH); ZAMBALDO, Claudio, 4070 Basel (CH)
(74) Representative: Vitra, Hermeto
(86) International application number: PCT/EP2022/056585
(87) International publication number: WO 2022/194800

(56) References cited:
- WO-A1-2020/005873
- WO-A1-2020/005877

## Description

The present invention relates to new organic compounds useful for therapy and/or prophylaxis in a mammal, and in particular to compounds that reduce the protein level of huntingtin (HTT) and which are useful in the treatment of Huntington's disease.

In particular, the present invention relates to a compound of formula (I) 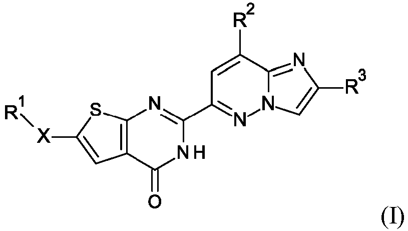 wherein
- X is: a bond or -O-;
- R¹ is: hydrogen, alkyl, cycloalkyl, aryl, heteroaryl or heterocycloalkyl wherein, each instance of cycloalkyl, aryl, heteroaryl and heterocycloalkyl is optionally substituted with one, two, three or four substituents independently selected from R⁴;
- R² is: hydrogen, cycloalkyl, alkenyl, cyano, amino, hydroxy, halogen, alkyl, haloalkyl, haloalkoxy or alkoxy;
- R³ is: hydrogen, alkyl, halogen or haloalkyl; and
- R⁴ is: halogen, alkyl, heterocycloalkyl, heterocycloalkylalkyl, alkylheterocycloalkyl, haloheterocycloalkyl, cycloalkyl, cycloalkylalkyl, alkylcycloalkyl, halocycloalkyl, cycloalkylamino, aryl, arylalkyl, alkylaryl, haloaryl, cyano, hydroxy, oxo, haloalkyl, alkylcarbonyl, alkoxy, haloalkoxy, alkoxyalkyl, alkoxycarbonyl, amino, alkylamino, dialkylamino, aminoalkyl, alkylaminoalkyl, dialkylaminoalkyl, aminoalkylamino, alkoxyalkylamino, alkylcarbonylamino, alkoxycarbonylamino, hydroxyalkyl, hydroxyalkoxyalkyl or hydroxyalkylamino;
or a pharmaceutically acceptable salt thereof.

Huntington's Disease (HD) is an inherited autosomal dominant neurodegenerative disease caused due to a CAG bases repeat expansion in the huntingtin (HTT) gene. Several lines of evidence indicate that the mutant HTT gene together with its gene product mHTT protein contributes to HD pathogenesis via a toxic gain of function mechanism.

The triplet repeat expansion in the exon 1 of the HTT gene translates into a polyglutamine repeat in the HTT protein which is prone to misfolding and aggregating in the cells. While the exact mechanisms of how mutant HTT disrupts cellular function is unclear, several processes ranging from interruption of RNA translation, toxic RNA species, protein aggregates, RNA translation, and stress granules have been implicated.

At a neural circuit level, HD has been shown to affects deep brains structures like the striatum as well as cortical regions to different extents. Seminal mouse genetic experiments coupled with human imaging experiments point to a key role of cortico-striatal connections in the pathogenicity of HD (Wang et al., "Neuronal targets of mutant huntingtin genetic reduction to ameliorate Huntington's disease pathogenesis in mice" Nature medicine 20.5 (2014): 536; Tabrizi et al.; "Potential endpoints for clinical trials in premanifest and early Huntington's disease in the TRACK-HD study: analysis of 24 month observational data." The Lancet Neurology 11.1 (2012): 42-53).

HD typically manifests around 30-50 years of age characterized by a multitude of symptoms spanning the motor, cognitive and affective domains eventually leading to death in 10-20 years after the onset of motor symptoms. CAG repeat length negatively correlates with age of onset of motor symptoms, however this only accounts for 50-70% of the variance in age of onset. In an effort to identify genetic modifiers of age of onset in HD, Lee et al. (2019, Huntington's disease onset is determined by length of uninterrupted CAG, not encoded polyglutamine, and is modified by DNA maintenance mechanisms. Bioarxiv doi: https://doi.org/10.1101/529768) conducted a large GWAS (genome-wide association study) that has uncovered additional genetic modifiers of age of onset.

Various mouse models have been characterized to model aspects of HD. The YAC128 mice expressing the full length mutant HTT transgene with 128 CAG repeats, BACHD mice expressing the full length mutant HTT genomic sequence with 97 CAG/CAA repeats, the R6/2 mice expressing exon 1 of the mutant human HTT gene with 110-135 CAG repeats). In addition to these mice that express the human transgene, there are also a series of mouse models, like the frequently used Q111, the Q175 knock in mice where the expanded repeats are knocked-in in the context of the mouse HTT locus.

There are currently no disease modifying therapies for Huntington's Disease while several are in development. The core disease process behind the symptomatology characterized by motor, cognitive and behavioral symptoms remains unmet by the various symptomatic treatments currently approved. Tetrabenazine and tiapride are currently approved for the treatment of motor symptoms namely HD-associated chorea. In addition, anticonvulsants, benzodiazepines, antidepressants, and antipsychotics are also used off-label to treat the motor, cognitive and psychiatric symptoms associated with HD.

Several therapeutic strategies targeting DNA and RNA are being investigated for HTT lowering (E. J. Wild, S. Tabrizi, Lancet Neurol. 2017 16(10): 837-847). HTT lowering is a promising therapeutic approach that aims to slow disease progression by getting at the core cause of Huntington's Disease. HTT lowering is thought to be transformative when treated in the pre-manifest or manifest stages of disease onset, thus preventing major neurodegenerative processes in the brain. However, the challenge lies in identifying the patients at the right disease stage, as age of onset is quite variable across the population (S. J. Tabrizi, R. Ghosh, B. R. Leavitt, Neuron, 2019, 102(4), 899).

The current clinical approaches are mainly based on antisense oligonucleotides (ASOs). In addition, a few allele specific lowering strategies such as SNP (single-nucleotide polymorphism) based ASO and zinc finger based gene editing approaches are investigated. While the use of small molecules to lower HTT expression has been postulated, this strategy hasn't yet been validated and none has proved successful so far.

WO2020/005877 discloses heteroaryl compounds for treating Huntington's disease.

Small molecules provide an opportunity to allow for HTT lowering in the brain as well as the periphery. In addition, a small molecule modality allows access to patient populations that are otherwise difficult to address with modalities like ASOs or gene therapy.

There is thus the need for new compounds capable of lowering mHTT.

The applicant has surprisingly found that the compounds of the invention are active in lowering mHTT and are therefore useful in the treatment of HD.

In the present description the term "alkyl", alone or in combination, signifies a straight-chain or branched-chain alkyl group with 1 to 8 carbon atoms, particularly a straight or branched-chain alkyl group with 1 to 6 carbon atoms and more particularly a straight or branched-chain alkyl group with 1 to 4 carbon atoms. Examples of straight-chain and branched-chain C1-C8 alkyl groups are methyl, ethyl, propyl, isopropyl, butyl, isobutyl, tert.-butyl, the isomeric pentyls, the isomeric hexyls, the isomeric heptyls and the isomeric octyls. Particular examples of "alkyl" are methyl, ethyl and isopropyl. Methyl is a further particular example of "alkyl" in the compound of formula (I).

In the present description the term "alkenyl", alone or in combination, signifies a straight-chain or branched-chain alkenyl group with 2 to 8 carbon atoms and further comprising at least one double bond, particularly a straight or branched-chain alkenyl group with 2 to 4 carbon atoms and further comprising at least one double bond. Particular examples of "alkenyl" are ethenyl, propenyl, isopropenyl, butenyl, isobutenyl and tertbutenyl.

The term "cycloalkyl", alone or in combination, signifies a cycloalkyl ring with 3 to 8 carbon atoms and particularly a cycloalkyl ring with 3 to 6 carbon atoms. Examples of cycloalkyl are cyclopropyl, cyclobutyl, cyclopentyl and cyclohexyl, cycloheptyl and cyclooctyl.

The term "aryl", alone or in combination, signifies an aromatic mono- or bicyclic ring system comprising 6 to 10 carbon ring atoms. Examples of "aryl" include, but are not limited to, phenyl and naphthyl.

The term "heteroaryl", alone or in combination, signifies an aromatic mono- or bicyclic ring system with 5 to 12 ring atoms, comprising 1, 2, 3 or 4 heteroatoms each independently selected from N, O and S, the remaining ring atoms being carbon. Examples of heteroaryl include, but are not limited to, furanyl, thiophenyl, 1H-pyrazolyl, 1H-imidazolyl, 1H-1,2,3-triazolyl, 4H-1,2,4-triazolyl, 1,2,4-oxadiazolyl, 1,3,4-oxadiazolyl, pyridinyl, pyridazinyl, pyrimidinyl, pyrazinyl, 1H-indolyl, 2H-indolyl, 1H-indazolyl, 2H-indazolyl, indolizinyl, benzofuranyl, 1H-benzimidazolyl, 1,3-benzoxazolyl, furo[2,3-b]pyridinyl, furo[2,3-c]pyridinyl, furo[3,2-b]pyridinyl, furo[3,2-c]pyridinyl, 1H-pyrrolo[2,3-b]pyridinyl, 1H-pyrrolo[2,3-c]pyridinyl, pyrrolo[1,2-a]pyrimidinyl, pyrrolo[1,2-a]pyrazinyl, pyrrolo[1,2-b]pyridazinyl, pyrazolo[1,5-a]pyridinyl, 1H-pyrazolo[4,3-b]pyridinyl, 2H-pyrazolo[4,3-b]pyridinyl, 2H-pyrazolo[4,3-c]pyridinyl, pyrazolo[1,5-a]pyrazinyl, pyrazolo[1,5-a]pyrimidinyl, imidazo[1,2-a]pyridinyl, imidazo[1,2-a]pyrimidinyl, imidazo[1,2-a]pyrazinyl, imidazo[1,2-b]pyridazinyl, imidazo[1,2-c]pyrimidinyl, imidazo[1,5-a]pyridinyl, imidazo[2,1-b][1,3]thiazolyl, imidazo[2,1-b][1,3,4]thiadiazolyl, [1,3]oxazolo[4,5-b]pyridinyl, [1,2,4]triazolo[1,5-a]pyridnyl, [1,2,4]triazolo[1,5-b]pyridazinyl, benzo[d]oxazolyl and quinolinyl.

The term "alkoxy" or "alkyloxy", alone or in combination, signifies a group of the formula alkyl-O- in which the term "alkyl" has the previously given significance. Particular examples of "alkoxy" are methoxy, ethoxy, n-propoxy, isopropoxy, n-butoxy, isobutoxy, sec-butoxy and tert.-butoxy.

The term "oxy", alone or in combination, signifies the -O- group.

The terms "halogen" or "halo", alone or in combination, signifies fluorine, chlorine, bromine or iodine and particularly fluorine, chlorine or bromine. One preferred example of halogen is fluorine. The term "halo", in combination with another group, if not otherwise specified, denotes the substitution of said group with at least one halogen, particularly substituted with one to five halogens, particularly one to four halogens, i.e. one, two, three or four halogens.

The term "haloalkyl", alone or in combination, denotes an alkyl group substituted with at least one halogen, particularly substituted with one to five halogens, particularly one to three halogens. Particular examples of "haloalkyl" are fluoromethyl, trifluoromethyl, difluoromethyl, fluoroethyl, fluoropropyl and fluorobutyl. A particular "haloalkyl" is trifluoromethyl.

The terms "hydroxyl" and "hydroxy", alone or in combination, signify the -OH group.

The term "cyano", alone or in combination, signifies the -CN group.

The term "carbonyl", alone or in combination, signifies the -C(O)- group.

The term "oxo", alone or in combination, signifies the =O group.

The term "amino", alone or in combination, signifies the primary amino group (-NH₂), the secondary amino group (-NH-), or the tertiary amino group (-N-).

The term "alkylamino", alone or in combination, signifies an alkyl group linked to a -NH- group. The term "dialkylamino", alone or in combination, signifies two alkyl groups linked to a -N- atom.

The term "heterocycloalkyl", alone or in combination, signifies a monocyclic or bicyclic saturated or monounsaturated ring system with 3 to 12 ring atoms, comprising 1, 2, 3 or 4 heteroatoms each independently selected from N, O and S, and the remaining ring atoms being carbon. In some particular embodiments the term "heterocycloalkyl", alone or in combination, can signify a monocyclic or bicyclic saturated or monounsaturated ring system with 5 to 10 ring atoms, comprising 1 or 2 nitrogen atoms and the remaining ring atoms being carbon. Particular "heterocycloalkyl" are piperazinyl, azetidinyl, morpholinyl, tetrahydropyranyl, tetrahydrofuranyl, 1,2,3,6-tetrahydropyridin-4-yl, 4,7-diazaspiro[2.5]octan-7-yl, (8aS)-3,4,6,7,8,8a-hexahydro-1H-pyrrolo[1,2-a]pyrazin-2-yl, (8aR)-3,4,6,7,8,8a-hexahydro-1H-pyrrolo[1,2-a]pyrazin-2-yl, 3,8-diazabicyclo[3.2.1]octan-8-yl, (1S,4S)-2,5-diazabicyclo[2.2.1]heptan-2-yl, 2,3,3a,4,6,6a-hexahydro-1H-pyrrolo[3,4-c]pyrrol-5-yl, pyrrolidinyl, 2,8-diazaspiro[4.5]decan-2-yl, 4-piperidyl and 4-azaspiro[2.5]octan-7-yl. Particular examples of "heterocycloalkyl" are azetidin-3-yl, 4-piperidyl and 4-azaspiro[2.5]octan-7-yl.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid, particularly hydrochloric acid, and organic acids such as formic acid, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein. In addition these salts may be prepared form addition of an inorganic base or an organic base to the free acid. Salts derived from an inorganic base include, but are not limited to, the sodium, potassium, lithium, ammonium, calcium, magnesium salts. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyamine resins. The compound of formula (I) can also be present in the form of zwitterions. Particularly preferred pharmaceutically acceptable salts of compounds of formula (I) are the salts formed with trifluoroacetic acid or hydrochloric acid.

If one of the starting materials or compounds of formula (I) contain one or more functional groups which are not stable or are reactive under the reaction conditions of one or more reaction steps, appropriate protecting groups (as described e.g. in "Protective Groups in Organic Chemistry" by T. W. Greene and P. G. M. Wuts, 3rd Ed., 1999, Wiley, New York) can be introduced before the critical step applying methods well known in the art. Such protecting groups can be removed at a later stage of the synthesis using standard methods described in the literature. Examples of protecting groups are tert-butoxycarbonyl (Boc), trityl (Trt), 2,4.dimethoxybenzyl (Dmb), 9-fluorenylmethyl carbamate (Fmoc), 2-trimethylsilylethyl carbamate (Teoc), carbobenzyloxy (Cbz) and p-methoxybenzyloxycarbonyl (Moz). A particular example of a protecting group is tert-butoxycarbonyl (Boc).

The compound of formula (I) can contain several asymmetric centers and can be present in the form of optically pure enantiomers, mixtures of enantiomers such as, for example, racemates, mixtures of diastereoisomers, diastereoisomeric racemates or mixtures of diastereoisomeric racemates.

The term "asymmetric carbon atom" means a carbon atom with four different substituents. According to the Cahn-Ingold-Prelog Convention an asymmetric carbon atom can be of the "R" or "S" configuration.

The invention thus also relates in particular to:
A compound according to the invention wherein X is a bond;
A compound according to the invention wherein X is -O-;
A compound according to the invention wherein R¹ is heterocylcoalkyl optionally substituted with one, two, three or four substituents independently selected from R⁴;
A compound according to the invention wherein R¹ is heterocylcoalkyl optionally substituted with one or two substituents independently selected from R⁴;
A compound according to the invention wherein R¹ is azetidin-3-yl, 4-piperidyl or 4-azaspiro[2.5]octan-7-yl, and wherein R¹ is optionally substituted with one or two substituents independently selected from R⁴;
A compound according to the invention wherein R¹ is 4-piperidyl or 4-azaspiro[2.5]octan-7-yl, and wherein R¹ is optionally substituted with one or two substituents independently selected from R⁴;
A compound according to the invention wherein R² is alkyl or haloalkyl;
A compound according to the invention wherein R² is methyl or trifluoromethyl;
A compound according to the invention wherein R³ is alkyl;
A compound according to the invention wherein R³ is methyl;
A compound according to the invention wherein R⁴ is halogen, alkyl or heterocycloalkyl;
A compound according to the invention wherein R⁴ is fluoro, methyl or pyrrolidinyl;
A compound according to the invention wherein R⁴ is halogen; and
A compound according to the invention wherein R⁴ is fluoro.

The invention further relates to a compound selected from
2-(2,8-dimethylimidazo[1,2-b]pyridazin-6-yl)-6-(4-piperidyl)-3H-thieno[2,3-d]pyrimidin-4-one;
2-(2,8-dimethylimidazo[1,2-b]pyridazin-6-yl)-6-[(3SR,4SR)-3-fluoro-4-piperidyl]-3H-thieno[2,3-d]pyrimidin-4-one;
6-(3,3-difluoro-4-piperidyl)-2-(2,8-dimethylimidazo[1,2-b]pyridazin-6-yl)-3H-thieno[2,3-d]pyrimidin-4-one;
6-(azetidin-3-yloxy)-2-(2,8-dimethylimidazo[1,2-b]pyridazin-6-yl)-3H-thieno[2,3-d]pyrimidin-4-one;
2-(2,8-dimethylimidazo[1,2-b]pyridazin-6-yl)-6-(4-piperidyloxy)-3H-thieno[2,3-d]pyrimidin-4-one;
6-(4-azaspiro[2.5]octan-7-yl)-2-(2,8-dimethylimidazo[1,2-b]pyridazin-6-yl)-3H-thieno[2,3-d]pyrimidin-4-one; and
2-[2-methyl-8-(trifluoromethyl)imidazo[1,2-b]pyridazin-6-yl]-6-(4-piperidyl)-3H-thieno[2,3-d]pyrimidin-4-one;
or a pharmaceutically acceptable salt thereof.

The invention further relates to a compound selected from
2-(2,8-dimethylimidazo[1,2-b]pyridazin-6-yl)-6-(4-piperidyl)-3H-thieno[2,3-d]pyrimidin-4-one;
2-(2,8-dimethylimidazo[1,2-b]pyridazin-6-yl)-6-[(3SR,4SR)-3-fluoro-4-piperidyl]-3H-thieno[2,3-d]pyrimidin-4-one;
2-(2,8-dimethylimidazo[1,2-b]pyridazin-6-yl)-6-(4-piperidyloxy)-3H-thieno[2,3-d]pyrimidin-4-one;
6-(4-azaspiro[2.5]octan-7-yl)-2-(2,8-dimethylimidazo[1,2-b]pyridazin-6-yl)-3H-thieno[2,3-d]pyrimidin-4-one; and
2-[2-methyl-8-(trifluoromethyl)imidazo[1,2-b]pyridazin-6-yl]-6-(4-piperidyl)-3H-thieno[2,3-d]pyrimidin-4-one;
or a pharmaceutically acceptable salt thereof.

The synthesis of the compound of formula (I) can, for example, be accomplished according to the following schemes. X and R¹-R⁴ are as defined above, unless otherwise specified.

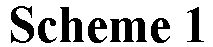

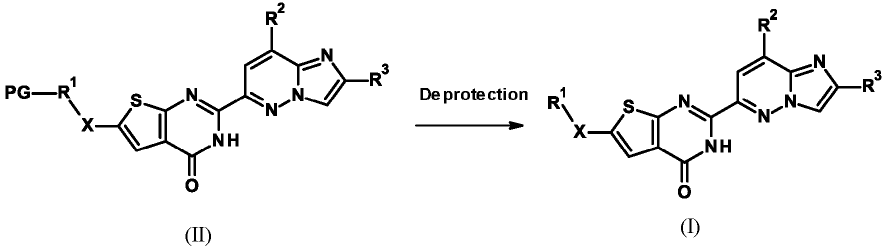

Compound of formula (I) can be prepared by methods known in the art e.g. deprotection of an intermediate of formula (II) with a protecting group (PG) by using an acid such as trifuloroacetic acid and a solvent such as dichloromethane. The syntheses of compounds of formulas (II) is outlined in scheme 2. General scheme 1 is hereinafter further illustrated by the general procedure C.

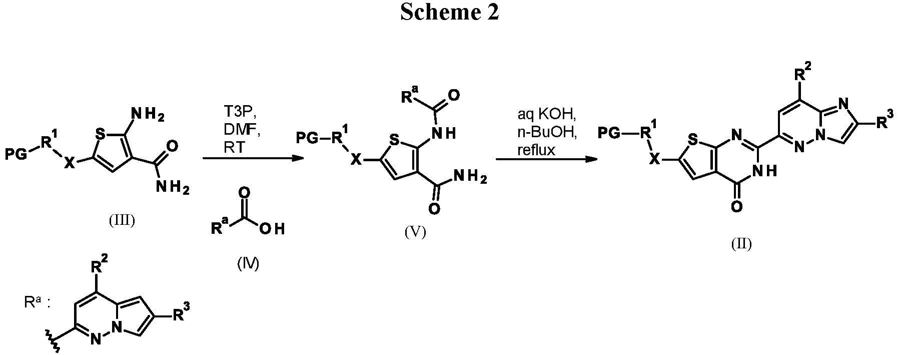

Compounds of formula (II) can be stepwise prepared by methods known in the art e.g. amide coupling of a carboxamide intermediate of formula (III) and an acid intemediate of formula (IV) and subsequent thermal cyclization of intermediates of formula (V) with aqueous inorganic base such as potassium hydroxide and a solvent such as n-butanol. Acid intemediates of formula (IV) are either commercially available or can be prepared by methods known in the art or described hereinafter. The syntheses of compounds of formulas (III) is outlined in scheme 3. General scheme 2 is hereinafter further illustrated by the general procedure B. 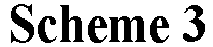 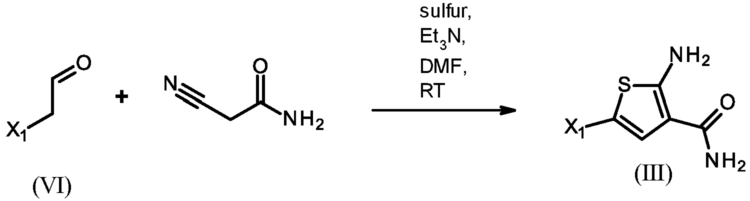 X₁ = X-R¹-PG :

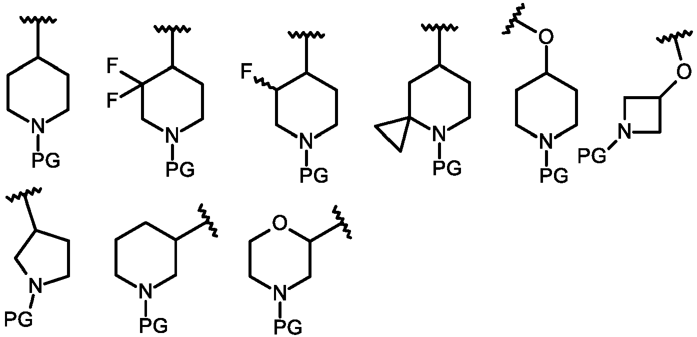

Carboxamide intermediates of formula (III) can be prepared by methods known in the art e.g. Gewald-type cyclization of an aldehyde of formula (VI) with cyanoacteamide and sulfur using a base such as triethylamine or morpholine and a solvent such as N,N-dimethylformamide or ethanol at room temperature up to 80 °C. General scheme 3 is hereinafter further illustrated by general procedure A.

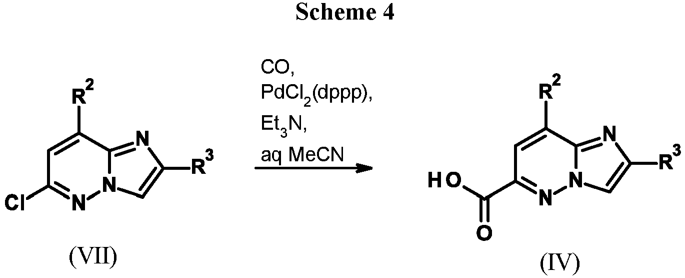

Acid intermediates of formula (IV) are either comercially available or can be prepared by methods known in the art e.g. Pd-catalyzed carbonylation of an intermediate of formula (VII) with carbonmonoxide in a solvent such as aqueous acetontrile a base such as triethylamine and a catalyst such as PdCl₂(dppp).

The invention thus also relates to a process for the preparation of a compound according to the invention, comprising at least one of the following steps:
(a) the reaction of a compound of formula (B1) 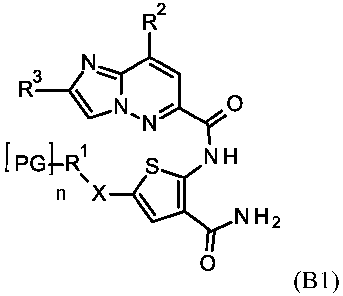 with a suitable base in presence of a suitable solvent, wherein n is 0 or 1, to arrive at a compound of formula (B2) 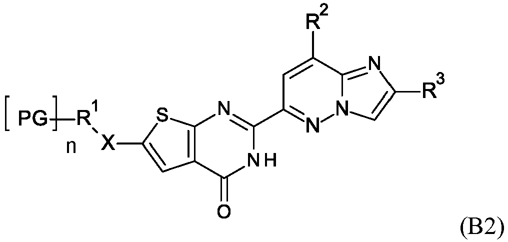
(b) the reaction of the compound of formula (B2), wherein n is 1, in a suitable solvent and in presence of an acid to yield the compound of formula (I) 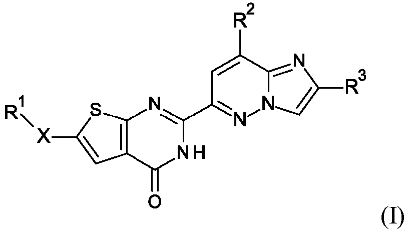 wherein in the process X, R¹, R², R³ and R⁴ are as defined above, and PG is a protecting group.
A mixture of the crude intermediate of formula (V) and a base such as 50% aqueous potassium hydroxide solution (2 eq) in a solvent such as n-butanol or THF is refluxed for 16-48 h. The reaction mixture is partitioned between a solvent such as ethyl acetate and aqueous sodium bicarbonate solution. The layers are separated. The aqueous layer is extracted with one or two portions of organic solvent. The combined organic layers are washed with one portion of brine, dried over anhydrous sodium sulfate and concentrated to dryness. Purification by flash-chromatography gives a thienopyrimidinone intermediate of formula (II).

The reaction of step (a) can be conveniently carried out in a solvent. The solvent can be for example THF, n-butanol or a mixture thereof;
In the reaction of step (a), the base can be for example NaOH or KOH, in particular KOH;
Convenient conditions for the reaction of step (a) are around 40 °C - 140 °C, particularly around 50 °C - 130 °C, more particularly around 60 °C - 120 °C;
Particular conditions for the reaction of step (a) are the use of KOH in THF, n-butanol or a mixture thereof at reflux for around 16 hrs - 48 hrs;
The reaction of step (b) can be conveniently carried out in a solvent. The solvent can be for example CH₂Cl₂ or 1,4-dioxane;
In the reaction of step (b) the acid can be for example 2,2,2-trifluoroacetic acid or HCl, in particular 2,2,2-trifluoroacetic acid;
In the reaction of step (b) the protecting group can be for example Boc, Trt or Dmb, in particular Boc;
Convenient conditions for the reaction of step (b) are around 0 °C - 100 °C, particularly around 5 °C - 70 °C, more particularly around 10 °C - 40 °C;

Particular conditions for the reaction of step (b) are the use of 2,2,2-trifluoroacetic acid in dichloromethane at RT for around 1 hrs - 72 hrs, in particular 1 hrs - 24 hrs.

The invention also relates to a compound according to the invention when manufactured according to a process of the invention.

The invention thus also relates in particular to:
A compound according to the invention for use as therapeutically active substance;
A pharmaceutical composition comprising a compound according to the invention and a therapeutically inert carrier;
A compound according to the invention for use in the treatment or prophylaxis of a neurodegenerative disease;
A compound according to the invention for use in the treatment or prophylaxis of Huntington's disease.

A certain embodiment of the invention relates to a pharmaceutical composition comprising the compound of formula (I) as described herein, or a pharmaceutically acceptable salt thereof, and a pharmaceutically acceptable auxiliary substance.

A certain embodiment of the invention relates to the compound of formula (I) asdescribed herein, or a pharmaceutically acceptable salt thereof, wherein at least one substituent comprises at least one radioisotope. Particular examples of radioisotopes are ²H, ³H, ¹³C, ¹⁴C and ¹⁸F.

Furthermore, the invention includes all optical isomers, i.e. diastereoisomers, diastereomeric mixtures, racemic mixtures, all their corresponding enantiomers and/or tautomers as well as their solvates, wherever applicable, of the compound of formula (I).

The compound of formula (I) may contain one or more asymmetric centers and can therefore occur as racemates, racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within this invention. The present invention is meant to encompass all such isomeric forms of these compounds. The independent syntheses of these diastereomers or their chromatographic separations may be achieved as known in the art by appropriate modification of the methodology disclosed herein. Their absolute stereochemistry may be determined by the x-ray crystallography of crystalline products or crystalline intermediates which are derivatized, if necessary, with a reagent containing an asymmetric center of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography.

In the embodiments, where optically pure enantiomers are provided, optically pure enantiomer means that the compound contains > 90 % of the desired isomer by weight, particularly > 95 % of the desired isomer by weight, or more particularly > 99 % of the desired isomer by weight, said weight percent based upon the total weight of the isomer(s) of the compound. Chirally pure or chirally enriched compounds may be prepared by chirally selective synthesis or by separation of enantiomers. The separation of enantiomers may be carried out on the final product or alternatively on a suitable intermediate.

Also an embodiment of the present invention is a compound of formula (I) as described herein, when manufactured according to any one of the described processes.
The compound of formula (I) or a pharmaceutically acceptable salt thereof can be used as a medicament (e.g. in the form of a pharmaceutical preparation). The pharmaceutical preparation can be administered internally, such as orally (e.g. in the form of tablets, coated tablets, dragées, hard and soft gelatin capsules, solutions, emulsions or suspensions), nasally (e.g. in the form of nasal sprays), rectally (e.g. in the form of suppositories) or topical ocularly (e.g. in the form of solutions, ointments, gels or water soluble polymeric inserts). However, the administration can also be effected parenterally, such as intramuscularly, intravenously, or intraocularly (e.g. in the form of sterile injection solutions).

The compound of formula (I) or a pharmaceutically acceptable salt thereof can be processed with pharmaceutically inert, inorganic or organic adjuvants for the production of tablets, coated tablets, dragées, hard gelatin capsules, injection solutions or topical formulations Lactose, corn starch or derivatives thereof, talc, stearic acid or its salts etc. can be used, for example, as such adjuvants for tablets, dragées and hard gelatin capsules.

Suitable adjuvants for soft gelatin capsules, are, for example, vegetable oils, waxes, fats, semi-solid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semi-solid or liquid polyols, etc.

Suitable adjuvants for topical ocular formulations are, for example, cyclodextrins, mannitol or many other carriers and excipients known in the art.

Moreover, the pharmaceutical preparation can contain preservatives, solubilizers, viscosity-increasing substances, stabilizers, wetting agents, emulsifiers, sweeteners, colorants, flavorants, salts for varying the osmotic pressure, buffers, masking agents or antioxidants. The pharmaceutical preparation can also contain still other therapeutically valuable substances.

The dosage can vary in wide limits and will be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should it be appropriate. In the case of topical administration, the formulation can contain 0.001% to 15% by weight of medicament and the required dose, which can be between 0.1 and 25 mg in can be administered either by single dose per day or per week, or by multiple doses (2 to 4) per day, or by multiple doses per week It will, however, be clear that the upper or lower limit given herein can be exceeded when this is shown to be indicated.

### Pharmaceutical Compositions

The compound of formula (I) or a pharmaceutically acceptable salt thereof can be used as a therapeutically active substance, e.g. in the form of a pharmaceutical preparation. The pharmaceutical preparation can be administered orally, e.g. in the form of tablets, coated tablets, dragées, hard and soft gelatin capsules, solutions, emulsions or suspensions. The administration can, however, also be effected rectally, e.g. in the form of suppositories, or parenterally, e.g. in the form of injection solutions.

The compound of formula (I) or a pharmaceutically acceptable salt thereof can be processed with pharmaceutically inert, inorganic or organic carriers for the production of a pharmaceutical preparation. Lactose, corn starch or derivatives thereof, talc, stearic acids or its salts and the like can be used, for example, as such carriers for tablets, coated tablets, dragées and hard gelatin capsules. Suitable carriers for soft gelatin capsules are, for example, vegetable oils, waxes, fats, semi-solid and liquid polyols and the like. Depending on the nature of the active substance no carriers are however usually required in the case of soft gelatin capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical preparation can, moreover, contain pharmaceutically acceptable auxiliary substances such as preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing a compound of formula (I) or a pharmaceutically acceptable salt thereof and a therapeutically inert carrier are also provided by the present invention, as is a process for their production, which comprises bringing a compound of formula (I) and/or pharmaceutically acceptable salts thereof and, if desired, one or more other therapeutically valuable substances into a galenical administration form together with one or more therapeutically inert carriers.

The dosage can vary within wide limits and will, have to be adjusted to the individual requirements in each particular case. In the case of oral administration the dosage for adults can vary from about 0.01 mg to about 1000 mg per day of a compound of general formula (I) or of the corresponding amount of a pharmaceutically acceptable salt thereof. The daily dosage may be administered as single dose or in divided doses and, in addition, the upper limit can also be exceeded when this is found to be indicated.

The following examples illustrate the present invention without limiting it, but serve merely as representative thereof. The pharmaceutical preparation conveniently contains about 1-500 mg, particularly 1-100 mg, of a compound of formula (I). Examples of compositions according to the invention are:

### Example A

Tablets of the following composition are manufactured in the usual manner:

**Table 1: possible tablet composition**

| ingredient | mg/tablet | | | |
|---|---|---|---|---|
| | 5 | 25 | 100 | 500 |
| Compound of formula (I) | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

### Example B-1

Capsules of the following composition are manufactured:

**Table 2: possible capsule ingredient composition**

| ingredient | mg/capsule | | | |
|---|---|---|---|---|
| | 5 | 25 | 100 | 500 |
| Compound of formula (I) | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

The compound of formula (I), lactose and corn starch are firstly mixed in a mixer and then in a comminuting machine. The mixture is returned to the mixer; the talc is added thereto and mixed thoroughly. The mixture is filled by machine into suitable capsules, e.g. hard gelatin capsules.

### Example B-2

Soft Gelatin Capsules of the following composition are manufactured:

**Table 3: possible soft gelatin capsule ingredient composition**

| ingredient | mg/capsule |
|---|---|
| Compound of formula (I) | 5 |
| Yellow wax | 8 |
| Hydrogenated Soya bean oil | 8 |
| Partially hydrogenated plant oils | 34 |
| Soya bean oil | 110 |
| Total | 165 |

**Table 4: possible soft gelatin capsule composition**

| ingredient | mg/capsule |
|---|---|
| Gelatin | 75 |
| Glycerol 85 % | 32 |
| Karion 83 | 8 (dry matter) |
| Titan dioxide | 0.4 |
| Iron oxide yellow | 1.1 |
| Total | 116.5 |

### Manufacturing Procedure

The compound of formula (I) is dissolved in a warm melting of the other ingredients and the mixture is filled into soft gelatin capsules of appropriate size. The filled soft gelatin capsules are treated according to the usual procedures.

### Example C

Suppositories of the following composition are manufactured:

**Table 5: possible suppository composition**

| ingredient | mg/supp. |
|---|---|
| Compound of formula (I) | 15 |
| Suppository mass | 1285 |
| Total | 1300 |

### Manufacturing Procedure

The suppository mass is melted in a glass or steel vessel, mixed thoroughly and cooled to 45°C. Thereupon, the finely powdered compound of formula (I) is added thereto and stirred until it has dispersed completely. The mixture is poured into suppository moulds of suitable size, left to cool; the suppositories are then removed from the moulds and packed individually in wax paper or metal foil.

### Example D

Injection solutions of the following composition are manufactured:

**Table 6: possible injection solution composition**

| ingredient | mg/injection solution. |
|---|---|
| Compound of formula (I) | 3 |
| Polyethylene Glycol 400 | 150 |
| acetic acid | q.s. ad pH 5.0 |
| water for injection solutions | ad 1.0 ml |

### Manufacturing Procedure

The compound of formula (I) is dissolved in a mixture of Polyethylene Glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

### Example E

Sachets of the following composition are manufactured:

**Table 7: possible sachet composition**

| ingredient | mg/sachet |
|---|---|
| Compound of formula (I) | 50 |
| Lactose, fine powder | 1015 |
| Microcrystalline cellulose (AVICEL PH 102) | 1400 |
| Sodium carboxymethyl cellulose | 14 |
| Polyvinylpyrrolidon K 30 | 10 |
| Magnesium stearate | 10 |
| Flavoring additives | 1 |
| Total | 2500 |

### Manufacturing Procedure

The compound of formula (I) is mixed with lactose, microcrystalline cellulose and sodium carboxymethyl cellulose and granulated with a mixture of polyvinylpyrrolidone in water. The granulate is mixed with magnesium stearate and the flavoring additives and filled into sachets.

### Examples

### Abbreviations:

Boc: tert-butyloxycarbonyl; BuOH: butanol; DCM: dichloromethane; DIBAL-H: diisobutylaluminium hydride; DMF: dimethylformamide; DMSO: dimethyl sulfoxide; dppp: 1,3-bis-(diphenylphosphino)-propan; EC₅₀: half maximal effective concentration; EtOAc: ethyl acetate; EtOH: ethanol; HTRF: homogeneous time resolved fluorescence ; LAH: lithium aluminium hydride; LCMS: liquid chromatography mass spectrometry; MeOH: methanol; MS: mass spectrometry; RP-HPLC: reversed phase-high performance liquid chromatography; RT: room temperature; SFC: supercritical fluid chromatography; TBME: *tert-butyl* methyl ether; TFA: trifluoroacetic acid; THF: tetrahydrofuran; T3P: propanephosphonic acid anhydride.

The following examples are provided for illustration of the invention. They should not be considered as limiting the scope of the invention, but merely as being representative thereof.

### Aldehydes of formula VI

### Aldehyde 1

### rac-(3S,4R)-3-Fluoro-4-(2-ketoethyl)piperidine-1-carboxylic acid tert-butyl ester

### a) (4E)-4-(2-ethoxy-2-keto-ethylidene)-3-fluoro-piperidine-1-carboxylic acid tert-butyl ester

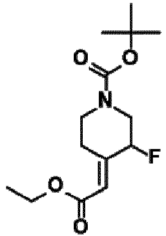

In a flame dried 500 mL 4-necked flask, equipped with a magnetic stirrer bar, dropping funnel and a thermometer, sodium hydride 60 % dispersion in mineral oil (2.0 g, 46.0 mmol, 1 eq) were added portion wise to a solution of triethyl phosphonoacetate (9.8 g, 8.8 mL, 43.7 mmol, 0.950 eq) in tetrahydrofuran, extra dry (200 mL) over 25 minutes at 0-5 °C. The ice-bath was removed and the mixture was stirred for 60 min. 3-fluoro-4-keto-piperidine-1-carboxylic acid tert-butyl ester (10 g, 46.0 mmol, 1 eq) dissolved in tetrahydrofuran, extra dry (100 mL) was added dropwise over 30 minutes. The reaction was quenched with saturated NH₄Cl solution (160 mL) and then partitioned between water (250 mL) and ethyl acetate (200 mL). The organic layer was separated. The aqueous layer was extracted with one 100-ml portion of ethyl acetate. The combined organic layers were washed with one 200-ml portion of brine, dried over anhydrous sodium sulfate, filtered and concentrated in vacuo. Purification by flash chromatography with heptane / ethyl acetate as eluent gave the title compound (8.51 g, 64%) as colourless oil. MS m/e: 188.0 ([M+H-C₅H₈O₂]⁺).

### b) (3S,4R)-4-(2-Ethoxy-2-keto-ethyl)-3-fluoro-piperidine-1-carboxylic acid tert-butyl ester and (3S,4S)-4-(2-Ethoxy-2-keto-ethyl)-3-fluoro-piperidine-1-carboxylic acid tert-butyl ester

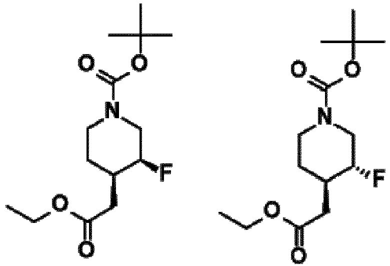

In a 2 L round-bottomed flask, a solution of (4E)-4-(2-ethoxy-2-keto-ethylidene)-3-fluoro-piperidine-1-carboxylic acid tert-butyl ester (8.51 g, 29.6 mmol, 1 eq) in ethyl acetate (567 mL) was degassed by three vacuum / argon cycles. Addition of palladium on charcoal 10% (3.15 g, 2.96 mmol, 0.100 eq). The reaction mixture was back-filled with hydrogen stirred under a hydrogen atmosphere (1 bar, RT) for 1 h. The catalyst was removed by filtration over a pad of Decalite. The filtrate was evaporated in vacuo. Purification by flash chromatography with methyl tert-butylether / heptane as gave (3S,4S)-4-(2-ethoxy-2-keto-ethyl)-3-fluoro-piperidine-1-carboxylic acid tert-butyl ester (2.18 g, 25 %) as a colourless viscous oil and (3S,4R)-4-(2-ethoxy-2-keto-ethyl)-3-fluoro-piperidine-1-carboxylic acid tert-butyl ester (5.87 g, 69 %) as a colourless viscous oil.

### c) (3S,4R)-3-Fluoro-4-(2-hydroxyethyl)piperidine-1-carboxylic acid tert-butyl ester

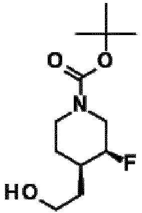

In a flame-dried 500 mL four necked flask, equipped with a thermometer and a dropping funnel (3 S,4R)-4-(2-ethoxy-2-keto-ethyl)-3-fluoro-piperidine-1-carboxylic acid tert-butyl ester (5.87 g, 20.3 mmol, 1 eq) was dissolved in tetrahydrofuran, extra dry (298 mL). 1 M lithium aluminum hydride in THF (20.3 mL, 20.3 mmol, 1 eq) was added over 30 minutes at 0-5 °C. Stirring was continued for 2 h. The reaction mixture was quenched by subsequent addition of water (0.770 mL), 2M aqueous NaOH (0.770 mL) and water (2.31 mL). The ice-bath was removed and the resulting slurry was stirred overnight. A white precipitate was formed, which was removed by filtration. The filter cake was washed with THF. The filtrate was evaporated to give the crude title compound (4.72 g, 94%) as colorless oil.

### d) (3S,4R)-3-Fluoro-4-(2-ketoethyl)piperidine-1-carboxylic acid tert-butyl ester

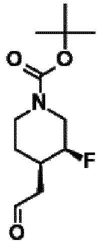

To a solution of (3 S,4R)-3-Fluoro-4-(2-hydroxyethyl)piperidine-1-carboxylic acid tert-butyl ester (1.83 g, 7.4 mmol, 1 eq) in dichloromethane (140 mL) was added 1,1,1-tris(acetyloxy)-1,1-dihydro-1,2-benzodioxol-3-(1H)-one (3.14 g, 7.4 mmol, 1 eq). The reaction mixture was stirred for 5 h at RT. TBME was added. The solids were removed by filtration. The filtrate was concentrated in vacuo. The residue was partitioned between ethyl acetate and water. The layers were separated. The aqueous layer was extracted with two portions of ethyl acetate. The combined organic layers were washed with one portion of brine, dried over anhydrous sodium sulfate and concentrated in vacuo to give the crude tilte compound (2 g) as white semisolid which was used in the next step without further purification.

### Aldehyde 2

### (3R,4R)-3-Fluoro-4-(2-ketoethyl)piperidine-1-carboxylic acid tert-butyl ester

### a) (3R,4R)-3-Fluoro-4-(2-hydroxyethyl)piperidine-1-carboxylic acid tert-butyl ester

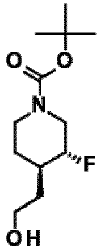

The title compound was prepared from (3R,4R)-4-(2-ethoxy-2-keto-ethyl)-3-fluoro-piperidine-1-carboxylic acid tert-butyl ester according to the procedure used for the synthesis of (3 S,4R)-3-fluoro-4-(2-hydroxyethyl)piperidine-1-carboxylic acid tert-butyl ester. MS m/e: 192.0 ([M+H-C₄H₈]⁺).

### b) (3R,4R)-3-Fluoro-4-(2-ketoethyl)piperidine-1-carboxylic acid tert-butyl ester

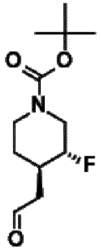

To a solution of (3R,4R)-3-fluoro-4-(2-hydroxyethyl)piperidine-1-carboxylic acid tert-butyl ester (771 mg, 3.12 mmol, 1 eq) in dichloromethane (31 mL) was added 1,1,1-tris(acetyloxy)-1,1-dihydro-1,2-benzodioxol-3-(1H)-one (1.32 g, 3.12 mmol, 1 eq) at RT. Stirring was continued for 2 h. The mixture was partitioned between DCM (30 ml) and 1M aqueous Na₂CO₃ sol (30 ml). The layers were separated. The aq layer was extracted with 50 mL of DCM. The combined organic layers were dried over sodium sulfate, filtered and concentrated in vacuo. The residue was triturated in diethyl ether (30 ml). The solids were removed by filtration. The filtrate was concentrated in vacuo to give the crude title compound (890 mg) as white solid, which was used in the next step without further purification. MS m/e: 190.1 ([M+H-C₄H₈]⁺).

### Aldehyde 3

### 3,3-Difluoro-4-(2-ketoethyl)piperidine-1-carboxylic acid tert-butyl ester

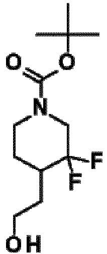

### a) 3,3-Difluoro-4-(2-hydroxyethyl)piperidine-1-carboxylic acid tert-butyl ester

To a solution of 4-(2-ethoxy-2-keto-ethyl)-3,3-difluoro-piperidine-1-carboxylic acid tert-butyl ester (260 mg, 0.846 mmol, 1 eq) in toluene (2 mL) at -78 °C, was added 1 M DIBAL-H in DCM (930 uL, 0.931 mmol, 1.1 eq) over a time of 10-15 min. The reaction mixture was stirred for 1.5 h. 133 ul water were added at - 78 °C. The reaction mixture was quenched by subsequent addition of water (0.133 mL), 1M aqueous NaOH (0.133 mL) and water (0.399 mL). The ice-bath was removed and the resulting slurry was stirred overnight. A white precipitate was formed, which was removed by filtration. The filter cake was washed with THF. The filtrate was concentrated in vacuo to afford a mixture of aldehyde and starting material. The mixture was dissolved in toluene (2 mL) and cooled to - 78 °C. 1 M DIBAL-H in DCM (1.70 mL, 1.69 mmol, 2 eq) was slowly added over a time of 10-15 min. The reaction mixture was stirred for 5 h at - 30 °C. The reaction mixture was quenched by subsequent addition of water (0.240 mL), 1M aqueous NaOH (0.240 mL) and water (0.720 mL). The ice-bath was removed and the resulting slurry was stirred overnight. A white precipitate was formed, which was removed by filtration. The filter cake was washed with THF. Purification by flash chromatography with n-heptane / ethyl acetate as eluent gave the title compound (127 mg, 56.58%) as colorless oil. MS m/e: 210.1 ([M+H]⁺).

### b) 3,3-Difluoro-4-(2-ketoethyl)piperidine-1-carboxylic acid tert-butyl ester

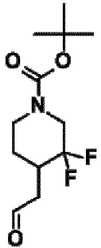

To a solution of DMSO (90 mg, 82 uL, 1.15 mmol, 2.4 eq) in dichloromethane (1.5 mL) was added oxalyl chloride (73 mg, 50 uL, 0.574 mmol, 1.2 eq) at - 78 °C. The mixture was stirred for 30 min at - 50 °C. The reaction mixture was cooled to - 78 °C. A solution of 3,3-difluoro-4-(2-hydroxyethyl)piperidine-1-carboxylic acid tert-butyl ester (127 mg, 0.479 mmol, 1 eq) in dichloromethane (1 mL) and slowly added. Stirring was continued for 1 h. Triethylamine (242 mg, 334 uL, 2.39 mmol, 5 eq) was added. The ice bath was removed after 15 min and the reaction mixture was allowed to warm up to RT. After 2 h the reaction mixture was partitioned between ethyl acetate and water. The layers were separated and the aqueous layer was extracted with two portions of ethyl acetate. The combined organic layers were washed with one portion of water / ammonium chloride and one portion of brine, dried over anhydrous sodium sulfate and concentrated in vacuo to give the crude title compound (132 mg) as colorless oil which was used in the net step without further purification. MS m/e: 164.0 ([M+H-C₅H₈O₂]⁺).

### Aldehyde 4

### 7-(2-Ketoethyl)-4-azaspiro[2.5]octane-4-carboxylic acid tert-butyl ester

### a) tert-Butyl (7E)-7-(2-ethoxy-2-oxo-ethylidene)-4-azaspiro[2.5]octane-4-carboxylate

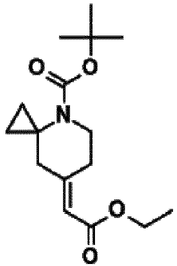

To a solution of NaH dispersion (194 mg, 4.44 mmol, 1 eq) in tetrahydrofuran (2 mL) was dropwise added a solution of triethyl phosphonoacetate (896 mg, 800 uL, 4.0 mmol, 0.900 eq) in tetrahydrofuran (2 mL) at 0 °C. The reaction mixture was allowed to warm up to RT and stirred for 15 min. The reaction mixture was cooled to 0 °C and a solution of 7-keto-4-azaspiro[2.5]octane-4-carboxylic acid tert-butyl ester (1 g, 4.44 mmol, 1 eq) in tetrahydrofuran (2 mL) was added. The reaction mixture was stirred for 3 h at RT. The reaction mixture was partitioned between ethyl acetate and 1 M aqueous NaOH. The layers were separated and the aqueous layer was extracted with two portions of ethyl acetate. The combined organic layers were washed with one portion of brine, dried over sodium sulfate, filtrated and concentrated in vacuo. Purification by flash chromatography with heptane / ethyl acetate as eluent gave the title compound (883 mg, 66%) as colorless oil with a purity of 98 %. MS m/e: 196.1 ([M+H-C₅H₈O₂]⁺).

### b) 7-(2-Ethoxy-2-keto-ethyl)-4-azaspiro[2.5]octane-4-carboxylic acid tert-butyl ester

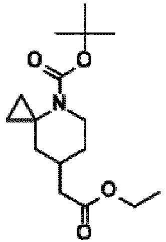

(7E)-7-(2-ethoxy-2-keto-ethylidene)-4-azaspiro[2.5]octane-4-carboxylic acid tert-butyl ester (883 mg, 2.93 mmol, 1 eq) was dissolved in ethyl acetate (10 mL). The atmosphere was exchanged with argon (3 x vacuum / argon). Palladium on activated charcoal (17 mg, 0.164 mmol, 0.056 eq) was added. The atmosphere was exchanged with hydrogen (3 x vacuum / hydrogen). The reaction mixture was stirred overnight at RT. The catalyst was removed by filtration over a satorious filter and concentrated in vacuo. Purification by flash chromatography with hepante / ethyl acetate as eluent gave the title compound (846 mg, 97.1%) as colorless oil. MS m/e: 198.2 ([M+H-C₅H₈O₂]⁺).

### c) 7-(2-Hydroxyethyl)-4-azaspiro[2.5]octane-4-carboxylic acid tert-butyl ester

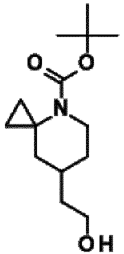

To a solution of 7-(2-ethoxy-2-keto-ethyl)-4-azaspiro[2.5]octane-4-carboxylic acid tert-butyl ester (846 mg, 2.85 mmol, 1 eq) in tetrahydrofuran (20 mL) was dropwise added 1 M LAH in THF (2.9 mL, 2.9 mmol, 1.01 eq) at 0 °C. The reaction mixture was stirred for 2 h at 0 °C. The reaction mixture was quenched by subsequent addition of water (0.15 ml), 4 N NaOH (0.15 ml) and water (0.45 ml). The solids were removed by filtration. The filtrate was concentrated in vacuo. The reaction mixture was partitioned between ethyl acetate and water and the layers were separated. The aqueous layer was extracted with two portions of ethyl acetate. The combined organic layers were washed with one portion of brine, dried over anhydrous sodium sulfate and concentrated in vacuo to give the crude title compound (730 mg, 100%) as light yellow oil which was used in the next step without further purification. MS m/e: 156.1 ([M+H-C₅H₈O₂]⁺).

### d) 7-(2-Ketoethyl)-4-azaspiro[2.5]octane-4-carboxylic acid tert-butyl ester

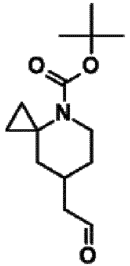

To a solution of 7-(2-hydroxyethyl)-4-azaspiro[2.5]octane-4-carboxylic acid tert-butyl ester (730 mg, 2.86 mmol, 1 eq) in dichloromethane (55 mL) was added 1,1,1-tris(acetyloxy)-1,1-dihydro-1,2-benzodioxol-3-(1H)-one (1.21 g, 2.86 mmol, 1 eq). The reaction mixture was stirred for 3 h at RT. TBME was added. The solids were removed by filtration. The filtrate was concentrated in vacuo. The residue was partitioned between ethyl acetate and water. The layers were separated. The aqueous layer was extracted with two portions of ethyl acetate. The combined organic layers were washed with one portion of brine, dried over anhydrous sodium sulfate and concentrated in vacuo. The crude product was triturated in ethyl acetate. The solids were removed by filtration. The filtrate was concentrated in vacuot to give the crude title compound as white semisolid. MS m/e: 154.1 ([M+H-C₅H₈O₂]⁺).

### Carboxamides of formula III

### General procedure A:

A mixture of an aldehyde of formula (VI) (1 eq), cyanoacetamide (1 eq), triethylamine (1 eq) and sulfur (1 eq) in N,N-dimethylformamide is stirred at room temperature for 16-48 h. The reaction mixture is partitioned between a solvent such as ethyl acetate and water. The layers are separated. The aqueous layer is extracted with one or two portions of organic solvent. The combined organic layers are washed with one portion of brine, dried over anhydrous sodium sulfate and concentrated to dryness. Purification by flash-chromatography gives a carboxamide of formula (III).

### Carboxamide 1

### 4-(5-Amino-4-carbamoyl-2-thienyl)piperidine-1-carboxylic acid tert-butyl ester

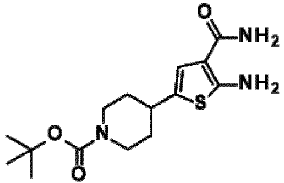

The title compound was obtained as brown oil with a purity of 93% by LCMS in 98% yield according to the general procedure A from 4-(2-ketoethyl)piperidine-1-carboxylic acid tert-butyl ester. MS m/e: 270.1 ([M+H]⁺)

### Carboxamide 2

### (3S,4S)-4-(5-Amino-4-carbamoyl-2-thienyl)-3-fluoro-piperidine-1-carboxylic acid tert-butyl ester

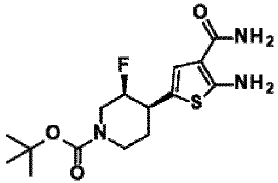

The title compound was obtained as off-white solid with a purity of 97% by LCMS in 17% yield according to the general procedure A from (3S,4R)-3-fluoro-4-(2-ketoethyl)piperidine-1-carboxylic acid tert-butyl ester. MS m/e: 342.3 ([M+H]⁺)

### Carboxamide 3

### 4-(5-Amino-4-carbamoyl-2-thienyl)-3,3-difluoro-piperidine-1-carboxylic acid tert-butyl ester

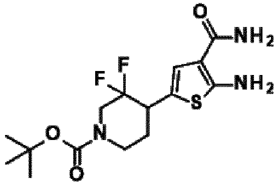

The title compound was obtained as orange solid with a purity of 99% by LCMS in 63% yield according to the general procedure A from 3,3-difluoro-4-(2-ketoethyl)piperidine-1-carboxylic acid tert-butyl ester. MS m/e: 360.3 ([M-H]⁻)

### Carboxamide 4

### 7-(5-Amino-4-carbamoyl-2-thienyl)-4-azaspiro[2.5]octane-4-carboxylic acid tert-butyl ester

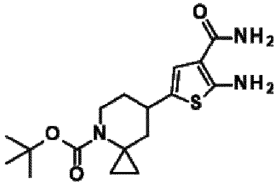

The title compound was obtained as orange solid with a purity of 98% by LCMS in 37% yield according to the general procedure A from 7-(2-ketoethyl)-4-azaspiro[2.5]octane-4-carboxylic acid tert-butyl ester. MS m/e: 350.2 ([M-H]⁻)

### Carboxamide 5

### 3-[(5-Amino-4-carbamoyl-2-thienyl)oxy]azetidine-1-carboxylic acid tert-butyl ester

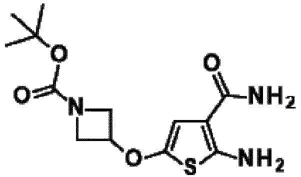

The title compound was obtained as brown oil with a purity of 95% by LCMS in 16% yield according to the general procedure A from 3-(2-ketoethoxy)azetidine-1-carboxylic acid tert-butyl ester. MS m/e: 312.2 ([M-H]⁻)

### Carboxamide 6

### 4-[(5-Amino-4-carbamoyl-2-thienyl)oxy]piperidine-1-carboxylic acid tert-butyl ester

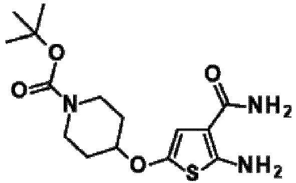

The title compound was obtained as brown oil with a purity of 95% by LCMS in 23% yield according to the general procedure A from 4-(2-ketoethoxy)piperidine-1-carboxylic acid tert-butyl ester. MS m/e: 340.3 ([M-H]⁻)

### Carboxamide 7

### 7-(5-Amino-4-carbamoyl-2-thienyl)-4-azaspiro[2.5]octane-4-carboxylic acid tert-butyl ester

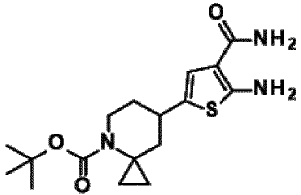

The title compound was obtained as orange solid with a purity of 98% by LCMS in 37% yield according to the general procedure A from 7-(2-ketoethyl)-4-azaspiro[2.5]octane-4-carboxylic acid tert-butyl ester. MS m/e: 350.2 ([M-H]⁻)

### Carboxamide 8

### (3R,4S)-4-(5-Amino-4-carbamoyl-2-thienyl)-3-fluoro-piperidine-1-carboxylic acid tert-butyl ester

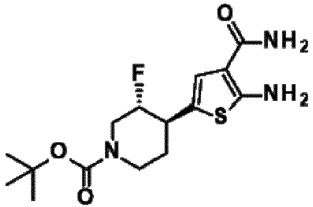

The title compound was obtained as light brown solid with a purity of 94% by LCMS in 39% yield according to the general procedure A from (3R,4R)-3-fluoro-4-(2-ketoethyl)piperidine-1-carboxylic acid tert-butyl ester. MS m/e: 342 ([M-H]⁻)

### Carboxamide 9

### (+)-(3S,4S)-4-(5-Amino-4-carbamoyl-2-thienyl)-3-fluoro-piperidine-1-carboxylic acid tert-butyl ester (enantiomer a)

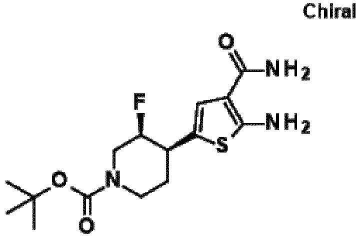

**and**

### Carboxamide 10

### (-)-(3R,4R)-4-(5-Amino-4-carbamoyl-2-thienyl)-3-fluoro-piperidine-1-carboxylic acid tert-butyl ester (enantiomer b)

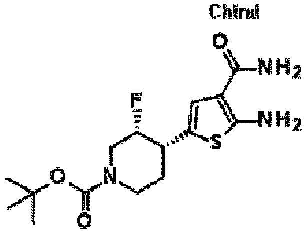

The two enantiomers which are putatively assigned were obtained from racemic (3S,4S)-4-(5-amino-4-carbamoyl-2-thienyl)-3-fluoro-piperidine-1-carboxylic acid tert-butyl ester by chiral SFC separation on a chiral Daicel IG column, 5 µm, 250 x 30 mm with 35% MeOH as co-solvent.
(+)-(3S,4S)-4-(5-Amino-4-carbamoyl-2-thienyl)-3-fluoro-piperidine-1-carboxylic acid tert-butyl ester enantiomer a was obtained as brown solid in 35% yield. MS m/e: 342.3 ([M-H]⁻), [α]D = +72.542 (c = 0.182 g / 100 ml, MeOH, 20 °C).
(-)-(3R,4R)-4-(5-Amino-4-carbamoyl-2-thienyl)-3-fluoro-piperidine-1-carboxylic acid tert-butyl ester enantiomer b was obtained as brown solid in 39% yield. MS m/e: 342.2 ([M-H]⁻), [α]D = -86.017 (c = 0.182 g / 100 ml, MeOH, 20 °C).

### Carboxamide 11

### (+)-(7S)-7-(5-Amino-4-carbamoyl-2-thienyl)-4-azaspiro[2.5]octane-4-carboxylic acid tert-butyl ester (enantiomer a)

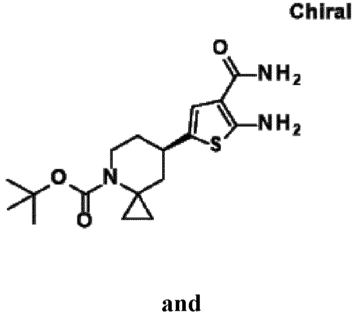

### Carboxamide 12

### (-)-(7R)-7-(5-amino-4-carbamoyl-2-thienyl)-4-azaspiro[2.5]octane-4-carboxylic acid tert-butyl ester (enantiomer b)

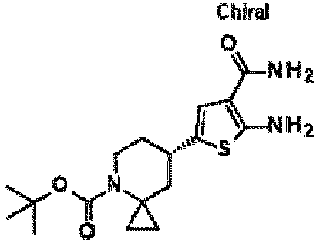

The two enantiomers which are putatively assigned were obtained from racemic 7-(5-amino-4-carbamoyl-2-thienyl)-4-azaspiro[2.5]octane-4-carboxylic acid tert-butyl ester by chiral SFC separation on a chiral CHIRALCEL OJ-H column, 5 µm, 250 x 20 mm, with 15% MeOH as co-solvent.
(+)-(7S)-7-(5-Amino-4-carbamoyl-2-thienyl)-4-azaspiro[2.5]octane-4-carboxylic acid tert-butyl ester enantiomer a was obtained as brown oil in 48% yield. MS m/e: 350.2 ([M-H]⁻), [α]D = +27.439 (c = 0.252 g / 100 ml, MeOH, 20 °C).
(-)-(7R)-7-(5-Amino-4-carbamoyl-2-thienyl)-4-azaspiro[2.5]octane-4-carboxylic acid tert-butyl ester enantiomer a was obtained as brown solid in 49% yield. MS m/e: 350.2 ([M-H]⁻), [α]D = -21.999 (c = 0.280 g / 100 ml, MeOH, 20 °C).

### Acid intermediates of formula IV

### Acid 1 2,8-Dimethylimidazo[1,2-b]pyridazine-6-carboxylic acid

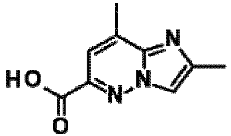

A mixture of 6-chloro-2,8-dimethyl-imidazo[1,2-b]pyridazine (10.0 g, 56.06 mmol) in acetonitrile (80 ml) and water (20 ml) was purged with argon. Addition of PdCl₂(dppp) (0.325 g, 0.551 mmol, 0.01 eq) and triethylamine (15.4 ml, 110.12 mmol, 2.0 eq). The mixture was carbonylated at 90 °C under an atmosphere of 60 bar carbonmonoxide for 72 h. The solids were removed by filtration and washed two 20-ml portions of acetonitrile. The filtrate was evaporated. The crude product was dissolved in dichloromethane (100 ml). After addition of 4M hydrogen chloride in 1,4-dioxane (13.8 ml, 55.08 mmol) the mixture was stirred for 3 h. The precipitate was collected by filtration, washed with two 20-ml portions of EtOH and dried in vacuo to give the title compound as off-white solid (9.46 g, 75%). MS m/e: 192.1 ([M+H]⁺)

### Acid 2

### 2-Methyl-8-(trifluoromethyl)imidazo[1,2-b]pyridazine-6-carboxylic acid

### a) 6-Chloro-2-methyl-8-(trifluoromethyl)imidazo[1,2-b]pyridazine

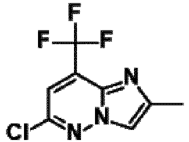

In a 10 mL round bottom flask, equipped with a magnetic strrer bar, reflux condenser and N₂-inlet bubbler, [6-chloro-4-(trifluoromethyl)pyridazin-3-yl]amine (94 mg, 0.476 mmol) and pyridinium p-toluene sulfonate (11.9 mg, 0.048 mmol) were combined with isopropanol (2 mL). 1-bromo-2,2-dimethoxy-propane (105 mg, 77 uL, 0.571 mmol, 1.2 eq) was added and the colorless solution was stirred 24 hours at 75 °C. The resulting dark-brown reaction mixture was cooled to RT and dilutes with EtOAc (10 mL) and washed with a saturated aqueous NaHCO₃-solution (10 mL). Organic layer was separated and dried over sodium sulfate, filtered off and concentrated in vacuo. Purification by column chromatography gave the title compound (46 mg, 34%) as light yellow solid. MS m/e: 236.1 ([M+H]⁺)

### b) 2-Methyl-8-(trifluoromethyl)imidazo[1,2-b]pyridazine-6-carboxylic acid

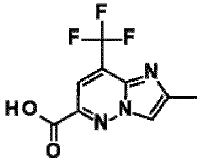

The title compound was obtained as light brown solid in 56% yield from 6-chloro-2-methyl-8-(trifluoromethyl)imidazo[1,2-b]pyridazine in analogy to the preparation of 2,8-dimethylimidazo[1,2-b]pyridazine-6-carboxylic acid. MS m/e: 246.1 ([M+H]⁺)

### Thienopyrimidinone intermediates of formula II

### General procedure B:

To a mixture of a carboxamide intermediate of formula (III) (1 eq) and an acid intermediate of formula (IV) (1.05-1.2 eq) in pyridine (0.3 M) is added an amide coupling reagent such as n-propylphosphonic acid anhydride (T3P) (1.2 eq) and stirring is continued at room temperature for 2-16 h. The reaction mixture is partitioned between a solvent such as ethyl acetate and aqueous sodium bicarbonate solution. The layers are separated. The aqueous layer is extracted with one or two portions of organic solvent. The combined organic layers are washed with one portion of brine, dried over anhydrous sodium sulfate and concentrated to dryness. A mixture of the crude intermediate of formula (V) and a base such as 50% aqueous potassium hydroxide solution (2 eq) in a solvent such as n-butanol or THF is refluxed for 16-48 h. The reaction mixture is partitioned between a solvent such as ethyl acetate and aqueous sodium bicarbonate solution. The layers are separated. The aqueous layer is extracted with one or two portions of organic solvent. The combined organic layers are washed with one portion of brine, dried over anhydrous sodium sulfate and concentrated to dryness. Purification by flash-chromatography gives a thienopyrimidinone intermediate of formula (II).

### Thienopyrimidinone 1

### 4-[2-(2,8-Dimethylimidazo[1,2-b]pyridazin-6-yl)-4-keto-3H-thieno[2,3-d]pyrimidin-6-yl]piperidine-1-carboxylic acid tert-butyl ester

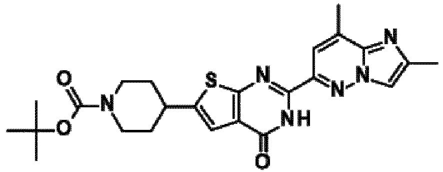

The title compound was obtained as off-white solid with a purity of 97% by LCMS according to the general procedure B from 4-(5-amino-4-carbamoyl-2-thienyl)piperidine-1-carboxylic acid tert-butyl ester and 2,8-dimethylimidazo[1,2-b]pyridazine-6-carboxylic acid. MS m/e: 481.3 ([M+H]⁺)

### Thienopyrimidinone 2

### (3SR,4SR)-4-[2-(2,8-Dimethylimidazo[1,2-b]pyridazin-6-yl)-4-keto-3H-thieno[2,3-d]pyrimidin-6-yl]-3-fluoro-piperidine-1-carboxylic acid tert-butyl ester

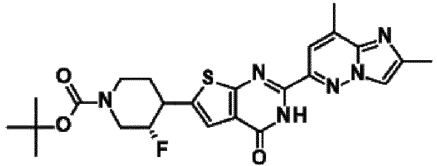

The title compound was obtained as light yellow solid with a purity of 94% by LCMS according to the general procedure B from (3SR,4SR)-4-(5-amino-4-carbamoyl-2-thienyl)-3-fluoro-piperidine-1-carboxylic acid tert-butyl ester and 2,8-dimethylimidazo[1,2-b]pyridazine-6-carboxylic acid. MS m/e: 499.3 ([M+H]⁺)

### Thienopyrimidinone 3

### 4-[2-(2,8-Dimethylimidazo[1,2-b]pyridazin-6-yl)-4-keto-3H-thieno[2,3-d]pyrimidin-6-yl]-3,3-difluoro-piperidine-1-carboxylic acid tert-butyl ester

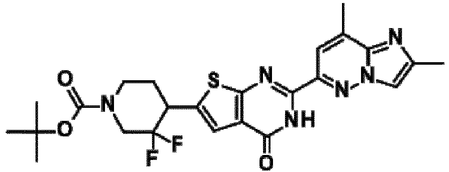

The title compound was obtained as light yellow solid with a purity of 94% by LCMS according to the general procedure B from 4-(5-amino-4-carbamoyl-2-thienyl)-3,3-difluoro-piperidine-1-carboxylic acid tert-butyl ester and 2,8-dimethylimidazo[1,2-b]pyridazine-6-carboxylic acid. MS m/e: 535.2 ([M+H]⁺)

### Thienopyrimidinone 4

### 3-[[2-(2,8-Dimethylimidazo[1,2-b]pyridazin-6-yl)-4-keto-3H-thieno|[2,3-d]pyrimidin-6-yl]oxy]azetidine-1-carboxylic acid tert-butyl ester

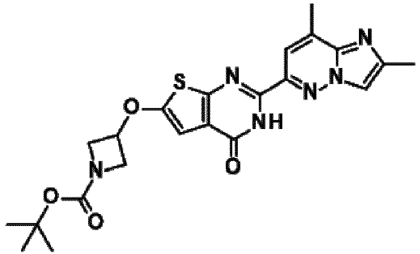

The title compound was obtained as off-white solid with a purity of 99% by LCMS according to the general procedure B from 3-[(5-amino-4-carbamoyl-2-thienyl)oxy]azetidine-1-carboxylic acid tert-butyl ester and 2,8-dimethylimidazo[1,2-b]pyridazine-6-carboxylic acid. MS m/e: 469.2 ([M+H]⁺)

### Thienopyrimidinone 5

### 4-[[2-(2,8-Dimethylimidazo[1,2-b]pyridazin-6-yl)-4-keto-3H-thieno[2,3-d]pyrimidin-6-yl]oxy]piperidine-1-carboxylic acid tert-butyl ester

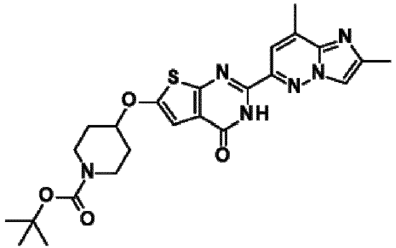

The title compound was obtained as yellow solid with a purity of 94% by LCMS according to the general procedure B from 4-[(5-amino-4-carbamoyl-2-thienyl)oxy]piperidine-1-carboxylic acid tert-butyl ester and 2,8-dimethylimidazo[1,2-b]pyridazine-6-carboxylic acid. MS m/e: 497.2 ([M+H]⁺)

### Thienopyrimidinone 6

### 7-[2-(2,8-Dimethylimidazo[1,2-b]pyridazin-6-yl)-4-keto-3H-thieno[2,3-d]pyrimidin-6-yl]-4-azaspiro[2.5]octane-4-carboxylic acid tert-butyl ester

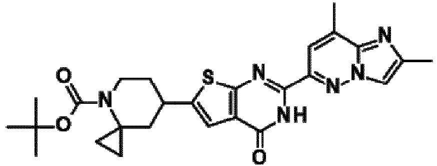

The title compound was obtained as light yellow solid with a purity of 83% by LCMS according to the general procedure B from 7-(5-amino-4-carbamoyl-2-thienyl)-4-azaspiro[2.5]octane-4-carboxylic acid tert-butyl ester and 2,8-dimethylimidazo[1,2-b]pyridazine-6-carboxylic acid. MS m/e: 507.3 ([M+H]⁺)

### Thienopyrimidinone 7

### 4-[4-Keto-2-[2-methyl-8-(trifluoromethyl)imidazo[1,2-b]pyridazin-6-yl]-3H-thieno[2,3-d]pyrimidin-6-yl]piperidine-1-carboxylic acid tert-butyl ester

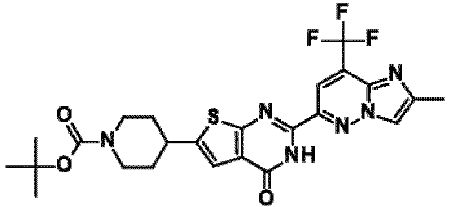

The title compound was obtained as yellow solid with a purity of 98% by LCMS according to the general procedure B from 4-(5-amino-4-carbamoyl-2-thienyl)piperidine-1-carboxylic acid tert-butyl ester and 2-methyl-8-(trifluoromethyl)imidazo[1,2-b]pyridazine-6-carboxylic acid. MS m/e: 553.2 ([M+H]⁺)

### Exemplified compounds of formula (I)

### General procedure C:

A solution of an N-BOC thienopyrimidinone derivative of general formula (II) (1 eq) and 2,2,2-trifluoroacetic acid (10-100 eq) in dichloromethane is stirred for 1-24 h at RT. Upon completition of the deprotection, the reaction mixture is concentrated in vacuo. The free base can be obtained by partitioning the TFA-salt between a base such as 1 M aqueous sodium bicarbonate solution or 1 M aqueous sodium carbonate solution and an organic solvent, e.g. ethyl acetate or dichloromethane. The layers are separated and the aqueous layer is extracted with two portions of the organic solvent. The combined organic layers are dried over anhydrous sodium sulfate, filtered and concentrated in vacuo. Purification by RP-HPLC, flash-chromatography or trituration from a suitable solvent such as ethyl acetate, ethanol or methanol gives rise to a compound of formula (I).

### Example 1

### 2-(2,8-Dimethylimidazo[1,2-b]pyridazin-6-yl)-6-(4-piperidyl)-3H-thieno[2,3-d]pyrimidin-4-one hydrogen chloride

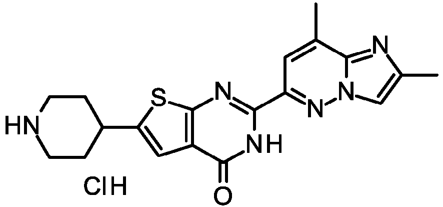

To a suspension of 4-[2-(2,8-dimethylimidazo[1,2-b]pyridazin-6-yl)-4-keto-3H-thieno[2,3-d]pyrimidin-6-yl]piperidine-1-carboxylic acid tert-butyl ester (50 mg, 0.105 mmol, 1 eq) in methanol (1 mL) was added 4 M aqueous HCl solution (324 mg, 270 uL, 1.08 mmol, 10.24 eq). The reaction mixture was stirred for three days at RT. The solids were collected by filtration, washed with ethyl acetate and dried in vacuo to give the title compound (28.9 mg, 65%) as light yellow solid. MS m/e: 381.3 ([M+H]⁺)

### Example 2

### 2-(2,8-Dimethylimidazo[1,2-b]pyridazin-6-yl)-6-[(3SR,4SR)-3-fluoro-4-piperidyl]-3H-thieno[2,3-d]pyrimidin-4-one

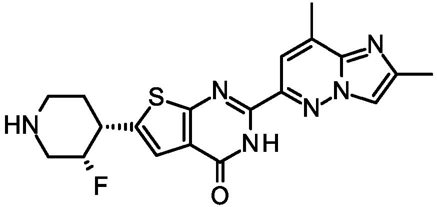

The title compound was obtained as light yellow solid with a purity of 97% by LCMS according to the general procedure C from (3SR,4SR)-4-[2-(2,8-dimethylimidazo[1,2-b]pyridazin-6-yl)-4-keto-3H-thieno[2,3-d]pyrimidin-6-yl]-3-fluoro-piperidine-1-carboxylic acid tert-butyl ester. MS m/e: 397.3 ([M+H]⁺)

### Example 3

### 6-(3,3-Difluoro-4-piperidyl)-2-(2,8-dimethylimidazo[1,2-b]pyridazin-6-yl)-3H-thieno[2,3-d]|pyrimidin-4-one

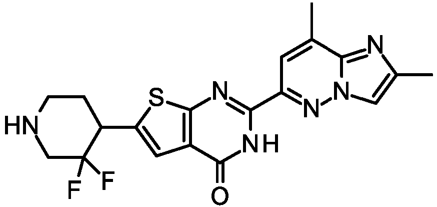

The title compound was obtained as white solid with a purity of 99% by LCMS according to the general procedure C from 4-[2-(2,8-dimethylimidazo[1,2-b]pyridazin-6-yl)-4-keto-3H-thieno[2,3-d]pyrimidin-6-yl]-3,3-difluoro-piperidine-1-carboxylic acid tert-butyl ester. MS m/e: 417.2 ([M+H]⁺)

### Example 4

### 6-(Azetidin-3-yloxy)-2-(2,8-dimethylimidazo[1,2-b]pyridazin-6-yl)-3H-thieno[2,3-d]pyrimidin-4-one

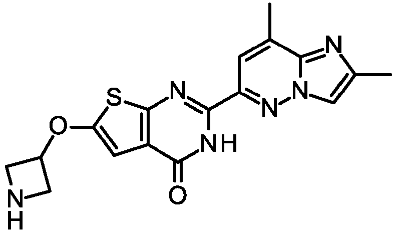

The title compound was obtained as light yellow solid with a purity of 98% by LCMS according to the general procedure C from 3-[[2-(2,8-dimethylimidazo[1,2-b]pyridazin-6-yl)-4-keto-3H-thieno[2,3-d]pyrimidin-6-yl]oxy]azetidine-1-carboxylic acid tert-butyl ester. MS m/e: 369.1 ([M+H]⁺)

### Example 5

### 2-(2,8-Dimethylimidazo[1,2-b]pyridazin-6-yl)-6-(4-piperidyloxy)-3H-thieno[2,3-d]pyrimidin-4-one

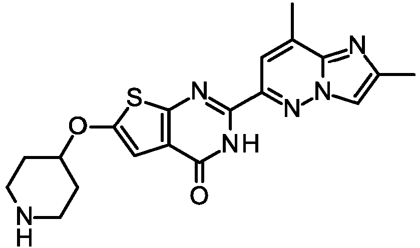

The title compound was obtained as yellow solid with a purity of 96% by LCMS according to the general procedure C from 4-[[2-(2,8-dimethylimidazo[1,2-b]pyridazin-6-yl)-4-keto-3H-thieno[2,3-d]pyrimidin-6-yl]oxy]piperidine-1-carboxylic acid tert-butyl ester. MS m/e: 397.2 ([M+H]⁺)

### Example 6

### 6-(4-Azaspiro[2.5]octan-7-yl)-2-(2,8-dimethylimidazo[1,2-b]pyridazin-6-yl)-3H-thieno[2,3-d]|pyrimidin-4-one

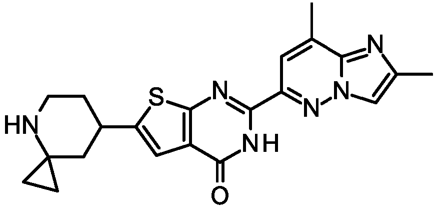

The title compound was obtained as off-white solid with a purity of 96% by LCMS according to the general procedure C from 7-[2-(2,8-dimethylimidazo[1,2-b]pyridazin-6-yl)-4-keto-3H-thieno[2,3-d]pyrimidin-6-yl]-4-azaspiro[2.5]octane-4-carboxylic acid tert-butyl ester. MS m/e: 407.2 ([M+H]⁺)

### Example 7

### 2-[2-Methyl-8-(trifluoromethyl)imidazo[1,2-b]pyridazin-6-yl]-6-(4-piperidyl)-3H-thieno[2,3-d]|pyrimidin-4-one

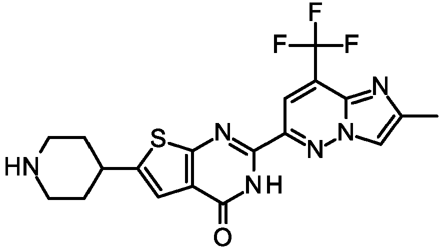

The title compound was obtained as yellow solid with a purity of 98% by LCMS according to the general procedure C from 4-[4-keto-2-[2-methyl-8-(trifluoromethyl)imidazo[1,2-b]pyridazin-6-yl]-3H-thieno[2,3-d]pyrimidin-6-yl]piperidine-1-carboxylic acid tert-butyl ester. MS m/e: 435.2 ([M+H]⁺)

### Example 8

### Homogeneous Time Resolved Fluorescence for HTT lowering

The HTRF assay was adapted from Weiss et al. (Analytical Biochemistry Volume 395, Issue 1, 1 December 2009, Pages 8-15 and Analytical Biochemistry Volume 410, 2011, Pages 304-306) to cells from GENEAe020-A cell line (https://hpscreg.eu/cell-line/GENEAe020-A).

Compounds were tested for the effect of mutant HTT levels in Huntington patient human cells (GENEAe020-A cell line) using Homogeneous Time Resolved Fluorescence (HTRF) directed towards mutant HTT protein (mHTT). The GENEAe020-A cell line was derived by Genea Biocells from human blastocysts of HD donors. After assessing viability, cells were plated into 384 well collagen coated plates in growth media. Once cells adhered, media was removed and test compounds dissolved in DMSO were diluted with buffer solution and added to the adherent cells. Controls included experiments with no cells, DMSO with no compound, and Hsp90 inhibitor control. Cells were incubated with compounds and controls for 48 hours. Then, the cells were lysed and transferred to an assay plate containing HTRF labeled monoclonal antibodies developed by Paul Patterson (Ko et al., Brain Research Bulletin, Volume 56, Numbers 3 and 4, 2001, Pages 319-329) which recognize specific areas of the HTT protein. The terbium labeled "donor" antibody (2B7) binds to the N-terminus of the HTT protein and the Alexa488 labeled "acceptor" antibody (MW1) is specific for the polyglutamine region of the protein. Binding of the acceptor labeled antibody is more efficient for the extended polyglutamine repeats of mutant HTT protein which translates into a signal boost which enables the specific measurement of mutant HTT protein level. The HTRF donor and acceptor detection reagents were incubated with the cell lysate and the ratio between the signals of the two fluorophores is indicative of the relative quantities of mHTT.

The results of this assay are provided in Table 1. Table 1 provides the EC₅₀ values for the reduction of mHTT obtained for particular examples of the present invention as measured by HTRF assay (data shown below is mean from three replicates).

| **Example** | **HTRF mHTT EC₅₀ (µM)** | **Example** | **HTRF mHTT EC₅₀ (µM)** |
|---|---|---|---|
| 1 | 0.050 | 4 | 4.695 |
| 2 | 0.205 | 5 | 0.282 |
| 3 | 2.269 | 6 | 0.203 |

## Claims

1. A compound of formula (I) 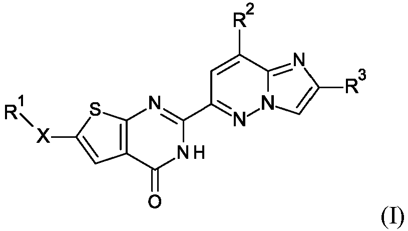 wherein
X is a bond or -O-;
R¹ is hydrogen, alkyl, cycloalkyl, aryl, heteroaryl or heterocycloalkyl wherein, each instance of cycloalkyl, aryl, heteroaryl and heterocycloalkyl is optionally substituted with one, two, three or four substituents independently selected from R⁴;
R² is hydrogen, cycloalkyl, alkenyl, cyano, amino, hydroxy, halogen, alkyl, haloalkyl, haloalkoxy or alkoxy;
R³ is hydrogen, alkyl, halogen or haloalkyl; and
R⁴ is halogen, alkyl, heterocycloalkyl, heterocycloalkylalkyl, alkylheterocycloalkyl, haloheterocycloalkyl, cycloalkyl, cycloalkylalkyl, alkylcycloalkyl, halocycloalkyl, cycloalkylamino, aryl, arylalkyl, alkylaryl, haloaryl, cyano, hydroxy, oxo, haloalkyl, alkylcarbonyl, alkoxy, haloalkoxy, alkoxyalkyl, alkoxycarbonyl, amino, alkylamino, dialkylamino, aminoalkyl, alkylaminoalkyl, dialkylaminoalkyl, aminoalkylamino, alkoxyalkylamino, alkylcarbonylamino, alkoxycarbonylamino, hydroxyalkyl, hydroxyalkoxyalkyl or hydroxyalkylamino;
or a pharmaceutically acceptable salt thereof.

2. A compound according to claim 1, wherein R¹ is heterocylcoalkyl optionally substituted with one or two substituents independently selected from R⁴.

3. A compound according to claim 1 or 2, wherein R¹ is azetidin-3-yl, 4-piperidyl or 4-azaspiro[2.5]octan-7-yl, and wherein R¹ is optionally substituted with one or two substituents independently selected from R⁴.

4. A compound according to any one of claims 1 to 3, wherein R¹ is 4-piperidyl or 4-azaspiro[2.5]octan-7-yl, and wherein R¹ is optionally substituted with one or two substituents independently selected from R⁴.

5. A compound according to any one of claims 1 to 4, wherein R² is alkyl or haloalkyl.

6. A compound according to any one of claims 1 to 5, wherein R² is methyl or trifluoromethyl.

7. A compound according to any one of claims 1 to 6, wherein R³ is alkyl.

8. A compound according to any one of claims 1 to 7, wherein R³ is methyl.

9. A compound according to any one of claims 1 to 8, wherein R⁴ is halogen.

10. A compound according to any one of claims 1 to 9, wherein R⁴ is fluoro.

11. A compound according to any one of claims 1 to 10, wherein X is a bond.

12. A compound according to any one of claims 1 to 11 selected from
2-(2,8-dimethylimidazo[1,2-b]pyridazin-6-yl)-6-(4-piperidyl)-3H-thieno[2,3-d]pyrimidin-4-one;
2-(2,8-dimethylimidazo[1,2-b]pyridazin-6-yl)-6-[(3SR,4SR)-3-fluoro-4-piperidyl]-3H-thieno[2,3-d]pyrimidin-4-one;
6-(3,3-difluoro-4-piperidyl)-2-(2,8-dimethylimidazo[1,2-b]pyridazin-6-yl)-3H-thieno[2,3-d]pyrimidin-4-one;
6-(azetidin-3-yloxy)-2-(2,8-dimethylimidazo[1,2-b]pyridazin-6-yl)-3H-thieno[2,3-d]pyrimidin-4-one;
2-(2,8-dimethylimidazo[1,2-b]pyridazin-6-yl)-6-(4-piperidyloxy)-3H-thieno[2,3-d]pyrimidin-4-one;
6-(4-azaspiro[2.5]octan-7-yl)-2-(2,8-dimethylimidazo[1,2-b]pyridazin-6-yl)-3H-thieno[2,3-d]pyrimidin-4-one; and
2-[2-methyl-8-(trifluoromethyl)imidazo[1,2-b]pyridazin-6-yl]-6-(4-piperidyl)-3H-thieno[2,3-d]pyrimidin-4-one;
or a pharmaceutically acceptable salt thereof.

13. A compound according to any one of claims 1 to 12 selected from
2-(2,8-dimethylimidazo[1,2-b]pyridazin-6-yl)-6-(4-piperidyl)-3H-thieno[2,3-d]pyrimidin-4-one;
2-(2,8-dimethylimidazo[1,2-b]pyridazin-6-yl)-6-[(3SR,4SR)-3-fluoro-4-piperidyl]-3H-thieno[2,3-d]pyrimidin-4-one;
2-(2,8-dimethylimidazo[1,2-b]pyridazin-6-yl)-6-(4-piperidyloxy)-3H-thieno[2,3-d]pyrimidin-4-one;
6-(4-azaspiro[2.5]octan-7-yl)-2-(2,8-dimethylimidazo[1,2-b]pyridazin-6-yl)-3H-thieno[2,3-d]pyrimidin-4-one; and
2-[2-methyl-8-(trifluoromethyl)imidazo[1,2-b]pyridazin-6-yl]-6-(4-piperidyl)-3H-thieno[2,3-d]pyrimidin-4-one;
or a pharmaceutically acceptable salt thereof.

14. A process for the preparation of a compound according to any one of claims 1 to 13, comprising at least one of the following steps:
(a) the reaction of a compound of formula (B1) 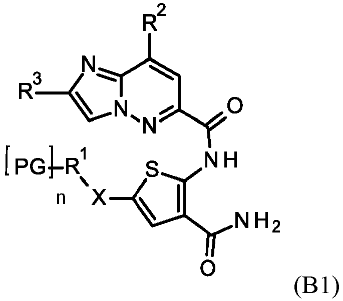 with a suitable base in presence of a suitable solvent, wherein n is 0 or 1, to arrive at a compound of formula (B2) 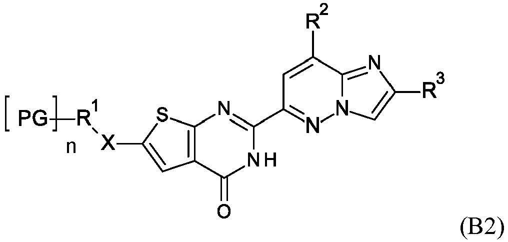
(b) the reaction of the compound of formula (B2), wherein n is 1, in a suitable solvent and in presence of an acid to yield the compound of formula (I) 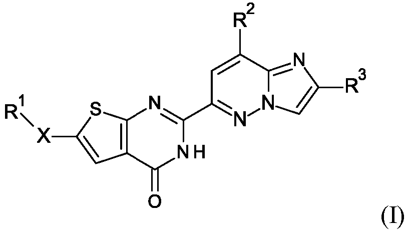 wherein in the process X, R¹, R², R³ and R⁴ are as defined in any one of claims 1 to 13, and PG is a protecting group.

15. A compound according to any one of claims 1 to 13 for use as therapeutically active substance.

16. A pharmaceutical composition comprising a compound according to any one of claims 1 to 13 and a therapeutically inert carrier.

17. A compound according to any one of claims 1 to 13 for use in the treatment or prophylaxis of a neurodegenerative disease, in particular Huntington's disease.

## Patentansprüche

1. Verbindung der Formel (I) 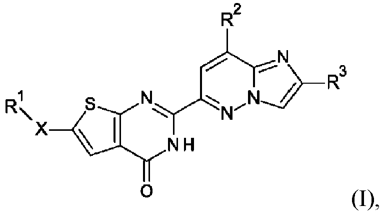 wobei
X eine Bindung oder -O- ist;
R¹ Wasserstoff, Alkyl, Cycloalkyl, Aryl, Heteroaryl oder Heterocycloalkyl ist, wobei jedes Beispiel für Cycloalkyl, Aryl, Heteroaryl und Heterocycloalkyl gegebenenfalls mit einem, zwei, drei oder vier Substituenten substituiert ist, die unabhängig voneinander aus R⁴ ausgewählt sind;
R² Wasserstoff, Cycloalkyl, Alkenyl, Cyano, Amino, Hydroxy, Halogen, Alkyl, Halogenalkyl, Halogenalkoxy oder Alkoxy ist;
R³ Wasserstoff, Alkyl, Halogen oder Halogenalkyl ist und
R⁴ Halogen, Alkyl, Heterocycloalkyl, Heterocycloalkylalkyl, Alkylheterocycloalkyl, Halogenheterocycloalkyl, Cycloalkyl, Cycloalkylalkyl, Alkylcycloalkyl, Halogencycloalkyl, Cycloalkylamino, Aryl, Arylalkyl, Alkylaryl, Halogenaryl, Cyano, Hydroxy, Oxo, Halogenalkyl, Alkylcarbonyl, Alkoxy, Halogenalkoxy, Alkoxyalkyl, Alkoxycarbonyl, Amino, Alkylamino, Dialkylamino, Aminoalkyl, Alkylaminoalkyl, Dialkylaminoalkyl, Aminoalkylamino, Alkoxyalkylamino, Alkylcarbonylamino, Alkoxycarbonylamino, Hydroxyalkyl, Hydroxyalkoxyalkyl oder Hydroxyalkylamino ist;
oder ein pharmazeutisch unbedenkliches Salz davon.

2. Verbindung nach Anspruch 1, wobei R¹ Heterocylcoalkyl ist, das gegebenenfalls mit einem oder zwei Substituenten substituiert ist, die unabhängig voneinander aus R⁴ ausgewählt sind.

3. Verbindung nach Anspruch 1 oder 2, wobei R¹ Azetidin-3-yl, 4-Piperidyl oder 4-Azaspiro[2.5]octan-7-yl ist und wobei R¹ gegebenenfalls mit einem oder zwei Substituenten substituiert ist, die unabhängig voneinander aus R⁴ ausgewählt sind.

4. Verbindung nach einem der Ansprüche 1 bis 3, wobei R¹ 4-Piperidyl oder 4-Azaspiro[2.5]octan-7-yl ist und wobei R¹ gegebenenfalls mit einem oder zwei Substituenten substituiert ist, die unabhängig voneinander aus R⁴ ausgewählt sind.

5. Verbindung nach einem der Ansprüche 1 bis 4, wobei R² Alkyl oder Halogenalkyl ist.

6. Verbindung nach einem der Ansprüche 1 bis 5, wobei R² Methyl oder Trifluormethyl ist.

7. Verbindung nach einem der Ansprüche 1 bis 6, wobei R³ Alkyl ist.

8. Verbindung nach einem der Ansprüche 1 bis 7, wobei R³ Methyl ist.

9. Verbindung nach einem der Ansprüche 1 bis 8, wobei R⁴ Halogen ist.

10. Verbindung nach einem der Ansprüche 1 bis 9, wobei R⁴ Fluor ist.

11. Verbindung nach einem der Ansprüche 1 bis 10, wobei X eine Bindung ist.

12. Verbindung nach einem der Ansprüche 1 bis 11, ausgewählt aus
2-(2,8-Dimethylimidazo[1,2-b]pyridazin-6-yl)-6-(4-piperidyl)-3H-thieno[2,3-d]pyrimidin-4-on;
2-(2,8-Dimethylimidazo[1,2-b]pyridazin-6-yl)-6-[(3SR,4SR)-3-fluor-4-piperidyl]-3H-thieno[2,3-d]pyrimidin-4-on;
6-(3,3-Difluor-4-piperidyl)-2-(2,8-dimethylimidazo[1,2-b]pyridazin-6-yl)-3H-thieno[2,3-d]pyrimidin-4-on;
6-(Azetidin-3-yloxy)-2-(2,8-dimethylimidazo[1,2-b]pyridazin-6-yl)-3H-thieno[2,3-d]pyrimidin-4-on;
2-(2,8-Dimethylimidazo[1,2-b]pyridazin-6-yl)-6-(4-piperidyloxy)-3H-thieno[2,3-d]pyrimidin-4-on;
6-(4-Azaspiro[2.5]octan-7-yl)-2-(2,8-dimethylimidazo[1,2-b]pyridazin-6-yl)-3H-thieno[2,3-d]pyrimidin-4-on und 2-[2-Methyl-8-(trifluormethyl)imidazo[1,2-b]pyridazin-6-yl]-6-(4-piperidyl)-3H-thieno[2,3-d]pyrimidin-4-on;
oder ein pharmazeutisch unbedenkliches Salz davon.

13. Verbindung nach einem der Ansprüche 1 bis 12, ausgewählt aus
2-(2,8-Dimethylimidazo[1,2-b]pyridazin-6-yl)-6-(4-piperidyl)-3H-thieno[2,3-d]pyrimidin-4-on;
2-(2,8-Dimethylimidazo[1,2-b]pyridazin-6-yl)-6-[(3SR,4SR)-3-fluor-4-piperidyl]-3H-thieno[2,3-d]pyrimidin-4-on;
2-(2,8-Dimethylimidazo[1,2-b]pyridazin-6-yl)-6-(4-piperidyloxy)-3H-thieno[2,3-d]pyrimidin-4-on;
6-(4-Azaspiro[2.5]octan-7-yl)-2-(2,8-dimethylimidazo[1,2-b]pyridazin-6-yl)-3H-thieno[2,3-d]pyrimidin-4-on und
2-[2-Methyl-8-(trifluormethyl)imidazo[1,2-b]pyridazin-6-yl]-6-(4-piperidyl)-3H-thieno[2,3-d]pyrimidin-4-on;
oder ein pharmazeutisch unbedenkliches Salz davon.

14. Verfahren zur Herstellung einer Verbindung nach einem der Ansprüche 1 bis 13, umfassend mindestens einen der folgenden Schritte:
(a) die Reaktion einer Verbindung der Formel (B1) 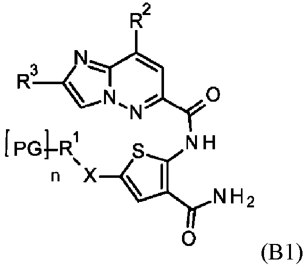 mit einer geeigneten Base in Gegenwart eines geeigneten Lösungsmittels, wobei n 0 oder 1 ist, um zu einer Verbindung der Formel (B2) zu gelangen 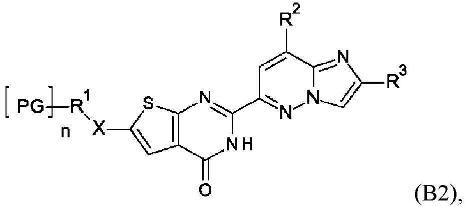
(b) die Reaktion der Verbindung der Formel (B2), wobei n 1 ist, in einem geeigneten Lösungsmittel und in Gegenwart einer Säure, was die Verbindung der Formel (I) ergibt 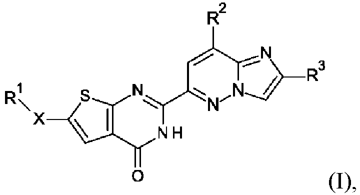 wobei in dem Verfahren X, R¹, R², R³ und R⁴ wie in einem der Ansprüche 1 bis 13 definiert sind und PG eine Schutzgruppe ist.

15. Verbindung nach einem der Ansprüche 1 bis 13 zur Verwendung als therapeutisch wirksame Substanz.

16. Pharmazeutische Zusammensetzung, umfassend eine Verbindung nach einem der Ansprüche 1 bis 13 und einen therapeutisch inerten Träger.

17. Verbindung nach einem der Ansprüche 1 bis 13 zur Verwendung bei der Behandlung oder Prophylaxe einer neurodegenerativen Krankheit, insbesondere der Huntington-Krankheit.

## Revendications

1. Composé de formule (I) 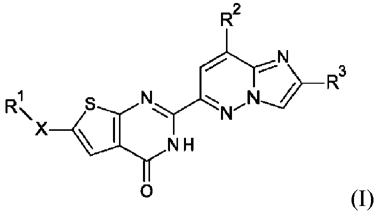 dans lequel
X représente une liaison ou -O- ;
R¹ représente un hydrogène, un alkyle, un cycloalkyle, un aryle, un hétéroaryle ou un hétérocycloalkyle dans lequel chaque occurrence du cycloalkyle, de l'aryle, de l'hétéroaryle et de l'hétérocycloalkyle est éventuellement substituée par un, deux, trois ou quatre substituants indépendamment choisis parmi R⁴ ;
R² représente un hydrogène, un cycloalkyle, un alcényle, un cyano, un amino, un hydroxy, un halogène, un alkyle, un halogénoalkyle, un halogénoalcoxy ou un alcoxy ;
R³ représente un hydrogène, un alkyle, un halogène ou un halogénoalkyle ; et
R⁴ représente un halogène, un alkyle, un hétérocycloalkyle, un hétérocycloalkylalkyle, un alkylhétérocycloalkyle, un halogénohétérocycloalkyle, un cycloalkyle, un cycloalkylalkyle, un alkylcycloalkyle, un halogénocycloalkyle, un cycloalkylamino, un aryle, un arylalkyle, un alkylaryle, un halogénoaryle, un cyano, un hydroxy, un oxo, un halogénoalkyle, un alkylcarbonyle, un alcoxy, un halogénoalcoxy, un alcoxyalkyle, un alcoxycarbonyle, un amino, un alkylamino, un dialkylamino, un aminoalkyle, un alkylaminoalkyle, un dialkylaminoalkyle, un aminoalkylamino, un alcoxyalkylamino, un alkylcarbonylamino, un alcoxycarbonylamino, un hydroxyalkyle, un hydroxyalcoxyalkyle ou un hydroxyalkylamino ;
ou un sel pharmaceutiquement acceptable de celui-ci.

2. Composé selon la revendication 1, dans lequel R¹ représente un hétérocycloalkyle éventuellement substitué par un ou deux substituants indépendamment choisis parmi R⁴.

3. Composé selon la revendication 1 ou 2, dans lequel R¹ représente un azétidin-3-yle, un 4-pipéridyle ou un 4-azaspiro[2.5]octan-7-yle, et dans lequel R¹ est éventuellement substitué par un ou deux substituants indépendamment choisis parmi R⁴.

4. Composé selon l'une quelconque des revendications 1 à 3, dans lequel R¹ représente un 4-pipéridyle ou un 4-azaspiro[2.5]octan-7-yle, et dans lequel R¹ est éventuellement substitué par un ou deux substituants indépendamment choisis parmi R⁴.

5. Composé selon l'une quelconque des revendications 1 à 4, dans lequel R² représente un alkyle ou un halogénoalkyle.

6. Composé selon l'une quelconque des revendications 1 à 5, dans lequel R² représente un méthyle ou un trifluorométhyle.

7. Composé selon l'une quelconque des revendications 1 à 6, dans lequel R³ représente un alkyle.

8. Composé selon l'une quelconque des revendications 1 à 7, dans lequel R³ représente un méthyle.

9. Composé selon l'une quelconque des revendications 1 à 8, dans lequel R⁴ représente un halogène.

10. Composé selon l'une quelconque des revendications 1 à 9, dans lequel R⁴ représente un fluor.

11. Composé selon l'une quelconque des revendications 1 à 10, dans lequel X représente une liaison.

12. Composé selon l'une quelconque des revendications 1 à 11 choisi parmi
la 2-(2,8-diméthylimidazo[1,2-b]pyridazin-6-yl)-6-(4-pipéridyl)-3H-thiéno[2,3-d]pyrimidin-4-one ;
la 2-(2,8-diméthylimidazo[1,2-b]pyridazin-6-yl)-6-[(3SR,4SR)-3-fluoro-4-pipéridyl]-3H-thiéno[2,3-d]pyrimidin-4-one ;
la 6-(3,3-difluoro-4-pipéridyl)-2-(2,8-diméthylimidazo[1,2-b]pyridazin-6-yl)-3H-thiéno[2,3-d]pyrimidin-4-one ;
la 6-(azétidin-3-yloxy)-2-(2,8-diméthylimidazo[1,2-b]pyridazin-6-yl)-3H-thiéno[2,3-d]pyrimidin-4-one ;
la 2-(2,8-diméthylimidazo[1,2-b]pyridazin-6-yl)-6-(4-pipéridyloxy)-3H-thiéno[2,3-d]pyrimidin-4-one ;
la 6-(4-azaspiro[2.5]octan-7-yl)-2-(2,8-diméthylimidazo[1,2-b]pyridazin-6-yl)-3H-thiéno[2,3-d]pyrimidin-4-one ; et
la 2-[2-méthyl-8-(trifluorométhyl)imidazo[1,2-b]pyridazin-6-yl]-6-(4-pipéridyl)-3H-thiéno[2,3-d]pyrimidin-4-one ;
ou un sel pharmaceutiquement acceptable de ceux-ci.

13. Composé selon l'une quelconque des revendications 1 à 12 choisi parmi
la 2-(2,8-diméthylimidazo[1,2-b]pyridazin-6-yl)-6-(4-pipéridyl)-3H-thiéno[2,3-d]pyrimidin-4-one ;
la 2-(2,8-diméthylimidazo[1,2-b]pyridazin-6-yl)-6-[(3SR,4SR)-3-fluoro-4-pipéridyl]-3H-thiéno[2,3-d]pyrimidin-4-one ;
la 2-(2,8-diméthylimidazo[1,2-b]pyridazin-6-yl)-6-(4-pipéridyloxy)-3H-thiéno[2,3-d]pyrimidin-4-one ;
la 6-(4-azaspiro[2.5]octan-7-yl)-2-(2,8-diméthylimidazo[1,2-b]pyridazin-6-yl)-3H-thiéno[2,3-d]pyrimidin-4-one ; et
la 2-[2-méthyl-8-(trifluorométhyl)imidazo[1,2-b]pyridazin-6-yl]-6-(4-pipéridyl)-3H-thiéno[2,3-d]pyrimidin-4-one ;
ou un sel pharmaceutiquement acceptable de ceux-ci.

14. Procédé de préparation d'un composé selon l'une quelconque des revendications 1 à 13, comprenant au moins l'une des étapes suivantes :
(a) la réaction d'un composé de formule (B1) 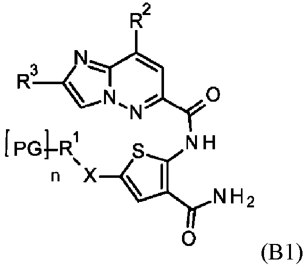 avec une base appropriée en présence d'un solvant approprié, dans lequel n représente 0 ou 1, pour arriver à un composé de formule (B2) 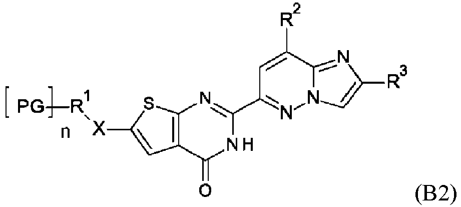
(b) la réaction du composé de formule (B2), dans lequel n représente 1, dans un solvant approprié et en présence d'un acide pour donner le composé de formule (I) 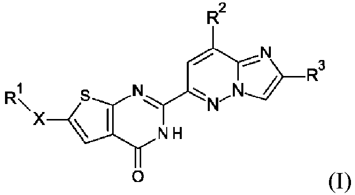 dans lequel dans le procédé X, R¹, R², R³ et R⁴ sont tels que définis dans l'une quelconque des revendications 1 à 13 et PG représente un groupe protecteur.

15. Composé selon l'une quelconque des revendications 1 à 13 pour une utilisation comme substance thérapeutiquement active.

16. Composition pharmaceutique comprenant un composé selon l'une quelconque des revendications 1 à 13 et un véhicule thérapeutiquement inerte.

17. Composé selon l'une quelconque des revendications 1 à 13 pour une utilisation dans le traitement ou la prophylaxie d'une maladie neurodégénérative, en particulier la maladie de Huntington.
